# EUROPÄISCHE PATENTSCHRIFT

(11) **EP 1 678 142 B1**
(45) Veröffentlichungstag und Bekanntmachung des Hinweises auf die Patenterteilung: **01.10.2014**
(21) Anmeldenummer: 04790293.7
(22) Anmeldetag: 12.10.2004
(51) Int. Cl.: C07D 231/14, C07D 231/16, C07D 277/56, C07D 327/06, C07D 333/38, C07D 307/68, C07D 285/06, C07D 213/82, C07D 241/24, C07C 211/46, A01N 43/56, A01N 43/40, A01N 43/78, A01N 43/32, A01N 43/08

(54) **HEXYLCARBOXANILIDE UND DEREN VERWENDUNG ZUR BEKÄMPFUNG VON UNERWÜNSCHTEN MIKROORGANISMEN**
HEXYL CARBOXANILIDES AND THEIR USE FOR CONTROLLING UNDESIRABLE MICRO-ORGANISMS
HEXYLCARBOXANILIDES ET LEUR UTILISATION POUR LUTTER CONTRE DES MICRO-ORGANISMES INDESIRABLES

(30) Priorität: 23.10.2003 DE 10349499; 07.11.2003 DE 10352082
(43) Veröffentlichungstag der Anmeldung: 12.07.2006
(73) Patentinhaber: Bayer CropScience AG, 40789 Monheim (DE)
(72) Erfinder: ELBE, Hans-Ludwig, 42329 Wuppertal (DE); GEBAUER, Olaf, 51377 Leverkusen (DE); GREUL, Jörg, Nico, 42799 Leichlingen (DE); HARTMANN, Benoit, 40764 Langenfeld (DE); WACHENDORFF-NEUMANN, Ulrike, 56566 Neuwied (DE); DAHMEN, Peter, 41470 Neuss (DE); KUCK, Karl-Heinz, 40764 Langenfeld (DE); DUNKEL, Ralf, 69001 Lyon (FR)
(86) Internationale Anmeldenummer: PCT/EP2004/011397
(87) Internationale Veröffentlichungsnummer: WO 2005/042493

(56) Entgegenhaltungen:
- WO-A-02/059086
- WO-A-03/010149

## Beschreibung

Die vorliegende Erfindung betrifft neue Hexylcarboxanilide, mehrere Verfahren zu deren Herstellung und deren Verwendung zur Bekämpfung von unerwünschten Mikroorganismen.

Es ist bereits bekannt, dass zahlreiche Carboxanilide fungizide Eigenschaften besitzen (vgl. z.B. WO 03/010149, WO 02/059086, WO 02/38542, WO 00/09482, EP-A 0 591 699, EP-A 0 589 301 und EP-A 0 545 099). So sind z.B. 5-Fluor-1,3-dimethyl-N-[2-(1,3,3-trimethylbutyl)phenyl]-1H-pyrazol-4-carboxamid aus WO 03/010149, N-Allyl-N-[2-(1,3-dimethylbutyl)phenyl]-1-methyl-3-(trifluormethyl)-1H-pyrazol-4-carboxamid aus WO 02/059086 und N-[2-(1,3-Dimethylbutyl)phenyl]-1-methyl-4-(trifluormethyl)-1H-pyrrol-3-carboxamid aus WO 02/38542 bekannt. Die Wirksamkeit dieser Stoffe ist gut, lässt aber bei niedrigen Aufwandmengen in manchen Fällen zu wünschen übrig.

Es wurden nun neue Hexylcarboxanilide der Formel (I) 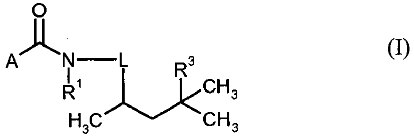 gefunden, in welcher
- L: für 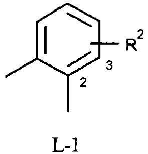 steht,
wobei die mit * markierte Bindung mit dem Amid verbunden ist,
- R¹: für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfmyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
(C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit
jeweils 1 bis 9 Fluor-, Chlor- und/oder Bromatomen; oder -C(=O)C(=O)R⁴, -CONR⁵R⁶ oder -CH₂NR⁷R⁸ steht,
- R²: für Wasserstoff, Fluor, Chlor, Methyl oder Trifluormethyl steht,
- R³: für Halogen, C₁-C₈-Alkyl oder C₁-C₈-Halogenalkyl steht,
- R⁴: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
- R⁵ und R⁶: unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
- R⁵ und R⁶: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁹ enthalten kann,
- R⁷ und R⁸: unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
- R⁷ und R⁸: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁹ enthalten kann,
- R⁹: für Wasserstoff oder C₁-C₆-Alkyl steht,
- A: für den Rest der Formel (A1) 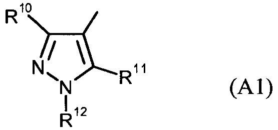 steht, in welcher
R¹⁰ für Wasserstoff, Hydroxy, Formyl, Cyano, Fluor, Chlor, Brom, Nitro, C₁-C₄-Alkyl, C₁-C₄-Alkoxy, C₁-C₄-Alkylthio, C₃-C₆-Cycloalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht,
R¹¹ für Wasserstoff, Chlor, Brom, Iod, Cyano, C₁-C₄-Alkyl, C₁-C₄-Alkoxy, C₁-C₄-Alkylthio, C₁-C₄-Halogenalkyl oder C₁-C₄-Halogenalkylthio mit jeweils 1 bis 5 Halogenatomen, steht und
R¹² für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder für Phenyl steht,

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Weiterhin wurde gefunden, dass man Hexylcarboxanilide der Formel (I) erhält, indem man
a) Carbonsäure-Derivate der Formel (II) 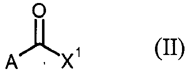 in welcher
   - A: die oben angegebenen Bedeutungen hat und
   - X¹: für Halogen oder Hydroxy steht,
   mit einem Anilin-Derivate der Formel (III) 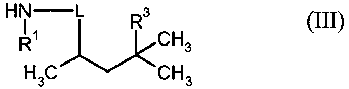 in welcher L, R¹ und R³ die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt,
   oder
b) Hexylcarboxanilide der Formel (I-a) 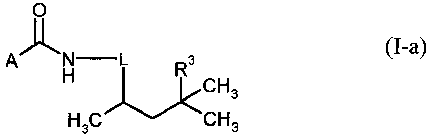 in welcher L, A und R³ die oben angegebenen Bedeutungen haben
   mit Halogeniden der Formel (IV)

   R^{1-A}-X² (IV)

   in welcher
   - X²: für Chlor, Brom oder Iod steht,
   - R^{1-A}: für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfmyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)-carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
   (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit jeweils 1 bis 9 Fluor-, Chlor- und/oder Bromatomen; oder -C(=O)C(=O)R⁴, -CONR⁵R⁶ oder -CH₂NR⁷R⁸ steht,
   wobei R⁴, R⁵, R⁶, R⁷ und R⁸ die oben angegebenen Bedeutungen haben,
in Gegenwart einer Base und in Gegenwart eines Verdünnungsmittels umsetzt.

Schließlich wurde gefunden, dass die neuen Hexylcarboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Die erfindungsgemäßen Hexylcarboxanilide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- R¹: steht bevorzugt für Wasserstoff, C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₆-Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R⁴, -CONR⁵R⁶ oder -CH₂NR⁷R⁸.
- R¹: steht besonders bevorzugt für Wasserstoff, Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl; Formyl, -CH₂-CHO, -(CH₂)₂-CHO, -CH₂-CO-CH₃, -CH₂-CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂-CO₂CH₃, -CH₂-CO₂CH₂CH₃, -CH₂-CO₂CH(CH₃)₂, -(CH₂)₂-CO₂CH₃, -(CH₂)₂-CO₂CH₂CH₃, -(CH₂)₂-CO₂CH(CH₃)₂, -CH₂-CO-CF₃, -CH₂-CO-CCl₃, -CH₂-CO-CH₂CF₃, -CH₂-CO-CH₂CCl₃, -(CH₂)₂-CO-CH₂CF₃, -(CH₂)₂-CO-CH₂CCl₃, -CH₂-CO₂CH₂CF₃, -CH₂-CO₂CF₂CF₃, -CH₂-CO₂CH₂CCl₃, -CH₂-CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CCl₃;
Methylcarbonyl, Ethylcarbonyl, n-Propylcarbonyl, iso-Propylcarbonyl, tert-Butylcarbonyl, Methoxycarbonyl, Ethoxycarbonyl, tert-Butoxycarbonyl, Cyclopropylcarbonyl; Trifluormethylcarbonyl, Trifluormethoxycarbonyl, oder -C(=O)C(=O)R⁵, -CONR⁶R⁷ oder -CH₂NR⁸R⁹.
- R¹: steht ganz besonders bevorzugt für Wasserstoff, Methyl, Methoxymethyl, Formyl, -CH₂-CHO, -(CH₂)₂-CHO, -CH₂-CO-CH₃, -CH₂-CO-CH₂CH₃, -CH₂-CO-CH(CH₃)₂, -C(=O)CHO, -C(=O)C(=O)CH₃, -C(=O)C(=O)CH₂OCH₃, -C(=O)CO₂CH₃, -C(=O)CO₂CH₂CH₃.
- R²: steht bevorzugt für Wasserstoff.
- R²: steht außerdem bevorzugt für Fluor, wobei Fluor besonders bevorzugt in 4-, 5- oder 6-Position, ganz besonders bevorzugt in 4- oder 6-Position, insbesondere in 4-Position des Anilidrestes steht [vgl. oben Formel (I)].
- R²: steht außerdem bevorzugt für Chlor, wobei Chlor besonders bevorzugt in 5-Position des Anilidrestes steht [vgl. oben Formel (I)]. Chlor steht außerdem besonders bevorzugt in 4-Position des Anilidrestes.
- R²: steht außerdem bevorzugt für Methyl, wobei Methyl besonders bevorzugt in 3-Position des Anilidrestes steht [vgl. oben Formel (I)].
- R²: steht außerdem bevorzugt für Trifluormethyl, wobei Trifluormethyl besonders bevorzugt in 4- oder 5-Position des Anilidrestes steht [vgl. oben Formel (I)].
- R³: steht bevorzugt für Fluor, Chlor, Brom, Iod, C₁-C₆-Alkyl, C₁-C₆-Halogenalkyl mit jeweils 1 bis 13 Fluor-, Chlor- und/oder Bromatomen.
- R³: steht besonders bevorzugt für Fluor, Chlor, Brom, Methyl, Ethyl, n-, iso-Propyl, n-, iso-, sec-, tert-Butyl oder für C₁-C₄-Halogenalkyl mit jeweils 1 bis 9 Fluor-, Chlor- und/oder Bromatomen.
- R³: steht ganz besonders bevorzugt für Fluor, Chlor, Methyl, Ethyl oder Trifluormethyl.
- R⁴: steht bevorzugt für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R⁴: steht besonders bevorzugt für Wasserstoff, Methyl, Ethyl, n- oder iso-Propyl, tert-Butyl, Methoxy, Ethoxy, n- oder iso-Propoxy, tert-Butoxy, Methoxymethyl, Cyclopropyl; Trifluormethyl, Trifluormethoxy.
- R⁵ und R⁶: stehen unabhängig voneinander bevorzugt für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R⁵ und R⁶: bilden außerdem gemeinsam mit dem Stickstoffatom, an das sie gebunden sind, bevorzugt einen gegebenenfalls einfach bis vierfach, gleich oder verschieden durch Halogen oder C₁-C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR⁹ enthalten kann.
- R⁵ und R⁶: stehen unabhängig voneinander besonders bevorzugt für Wasserstoff, Methyl, Ethyl, n-oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁵ und R⁶: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R⁹ substituiert sein kann.
- R⁷ und R⁸: stehen unabhängig voneinander bevorzugt für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor- und/oder Bromatomen.
- R⁷ und R⁸: bilden außerdem gemeinsam mit dem Stickstoffatom, an das sie gebunden sind, bevorzugt einen gegebenenfalls einfach oder mehrfach, gleich oder verschieden durch Halogen oder C₁-C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR⁹ enthalten kann.
- R⁷ und R⁸: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁷ und R⁸: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R⁹ substituiert sein kann.
- R⁹: steht bevorzugt für Wasserstoff oder C₁-C₄-Alkyl.
- R⁹: steht besonders bevorzugt für Wasserstoff, Methyl, Ethyl, n- oder iso-Propyl, n-, iso-, sec- oder tert-Butyl.
- R¹⁰: steht bevorzugt für Wasserstoff, Hydroxy, Formyl, Cyano, Fluor, Chlor, Brom, Methyl, Ethyl, iso-Propyl, Methoxy, Ethoxy, Methylthio, Ethylthio, Cyclopropyl, C₁-C₂-Halogenalkyl, C₁-C₂-Halogenalkoxy mit jeweils 1 bis 5 Fluor, Chlor und/oder Bromatomen, Trifluormethylthio, Difluormethylthio, Aminocarbonyl, Aminocarbonylmethyl oder Aminocarbonylethyl.
- R¹⁰: steht besonders bevorzugt für Wasserstoff, Hydroxy, Formyl, Fluor, Chlor, Brom, Methyl, Ethyl, iso-Propyl, Methoxy, Ethoxy, Monofluormethyl, Monofluorethyl, Difluormethyl, Trifluormethyl, Difluorchlormethyl, Trichlormethyl, Dichlormethyl, Pentafluorethyl, Cyclopropyl, Methoxy, Ethoxy, Trifluormethoxy, Difluormethoxy, Trichlormethoxy, Methylthio, Ethylthio, Trifluormethylthio oder Difluormethylthio.
- R¹⁰: steht ganz besonders bevorzugt für Wasserstoff, Hydroxy, Formyl, Fluor, Chlor, Brom, Methyl, Ethyl, iso-Propyl, Methoxy, Cyclopropyl, Monofluormethyl, Monofluorethyl, Difluormethyl, Dichlormethyl, Trifluormethyl, Difluorchlormethyl, Trichlormethyl, -CHFCH₃ oder Difluormethoxy.
- R¹⁰: steht insbesondere bevorzugt für Wasserstoff, Hydroxy, Formyl, Chlor, Methyl, Ethyl, Methoxy, Cyclopropyl, Monofluormethyl, Difluormethyl, Dichlormethyl, Trifluormethyl, -CHFCH₃ oder Difluormethoxy.
- R¹¹: steht bevorzugt für Wasserstoff, Chlor, Brom, Iod, Methyl, Ethyl, Methoxy, Ethoxy, Methylthio, Ethylthio, C₁-C₂-Halogenalkyl mit 1 bis 5 Fluor, Chlor und/oder Bromatomen,
- R¹¹: steht besonders bevorzugt für Wasserstoff, Chlor, Brom, Iod, Methyl oder -CHFCH₃.
- R¹¹: steht ganz besonders bevorzugt für Wasserstoff, Chlor, Methyl oder -CHFCH₃.
- R¹²: steht bevorzugt für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl.
- R¹²: steht besonders bevorzugt für Wasserstoff, Methyl, Ethyl, iso-Propyl, Trifluormethyl, Difluormethyl, Hydroxymethyl, Hydroxyethyl oder Phenyl.
- R¹²: steht ganz besonders bevorzugt für Wasserstoff, Methyl, Trifluormethyl oder Phenyl.
- R¹²: steht insbesondere bevorzugt für Methyl.
- R¹³ und R¹⁴: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R¹³ und R¹⁴: stehen unabhängig voneinander besonders bevorzugt für Wasserstoff, Fluor, Chlor, Brom, Methyl, Ethyl, Difluormethyl, Trifluormethyl, Difluorchlormethyl oder Trichlormethyl.
- R¹³ und R¹⁴: stehen unabhängig voneinander ganz besonders bevorzugt für Wasserstoff, Fluor, Chlor, Brom oder Methyl.
- R¹³ und R¹⁴: stehen insbesondere bevorzugt jeweils für Wasserstoff.
- R¹⁵: steht bevorzugt für Fluor, Chlor, Brom, Iod, Cyano, Methyl, Ethyl, C₁-C₂-Halogenalkyl oder C₁-C₂-Halogenalkoxy mit jeweils 1 bis 5 Fluor, Chlor und/oder Bromatomen.
- R¹⁵: steht besonders bevorzugt für Fluor, Chlor, Brom, Iod, Cyano, Methyl, Trifluormethyl, Trifluormethoxy, Difluormethoxy, Difluorchlormethoxy oder Trichlormethoxy.
- R¹⁵: steht ganz besonders bevorzugt für Fluor, Chlor, Brom, Iod, Methyl, Trifluormethyl oder Trifluormethoxy.
- R¹⁵: steht insbesondere bevorzugt für Chlor oder Methyl.
- R¹⁶ und R¹⁷: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R¹⁶ und R¹⁷: stehen unabhängig voneinander besonders bevorzugt für Wasserstoff, Fluor, Chlor, Brom, Methyl, Ethyl, Difluormethyl, Trifluormethyl, Difluorchlormethyl oder Trichlormethyl.
- R¹⁶ und R¹⁷: stehen unabhängig voneinander ganz besonders bevorzugt für Wasserstoff, Fluor, Chlor, Brom oder Methyl.
- R¹⁶ und R¹⁷: stehen insbesondere bevorzugt jeweils für Wasserstoff.
- R¹⁸: steht bevorzugt für Wasserstoff, Methyl, Ethyl oder C₁-C₂-Halogenalkyl mit 1 bis 5 Fluor, Chlor und/oder Bromatomen.
- R¹⁸: steht besonders bevorzugt für Wasserstoff, Methyl oder Trifluormethyl.
- R¹⁸: steht ganz besonders bevorzugt für Methyl.
- R¹⁹: steht bevorzugt für Fluor, Chlor, Brom, Iod, Hydroxy, Cyano, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen.
- R¹⁹: steht besonders bevorzugt für Fluor, Chlor, Brom, Iod, Hydroxy, Cyano, Methyl, Ethyl, n-Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Difluormethylthio, Trifluormethylthio, Trifluormethoxy, Difluormethoxy, Difluorchlormethoxy oder Trichlormethoxy.
- R¹⁹: steht ganz besonders bevorzugt für Fluor, Chlor, Brom, Iod, Hydroxy, Methyl, Trifluormethyl, Difluormethyl oder Trichlormethyl.
- R²⁰: steht bevorzugt für Wasserstoff, Fluor, Chlor, Brom, Iod, Cyano, C₁-C₄-Alkyl, Methoxy, Ethoxy, Methylthio, Ethylthio, C₁-C₂-Halogenalkyl oder C₁-C₂-Halogenalkoxy mit jeweils 1 bis 5 Fluor, Chlor und/oder Bromatomen, C₁-C₂-Alkylsulfinyl oder C₁-C₂-Alkylsulfonyl.
- R²⁰: steht besonders bevorzugt für Wasserstoff, Fluor, Chlor, Brom, Iod, Cyano, n-Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Trifluormethoxy, Difluormethoxy, Difluorchlormethoxy, Trichlormethoxy, Methylsulfinyl oder Methylsulfonyl.
- R²⁰: steht ganz besonders bevorzugt für Wasserstoff, Fluor, Chlor, Brom, Iod, n-Propyl, iso-Propyl, n-Butyl, iso-Butyl, sec-Butyl, tert-Butyl, Trifluormethyl, Difluormethyl, Trichlormethyl, Methylsulfinyl oder Methylsulfonyl.
- R²⁰: steht insbesondere bevorzugt für Wasserstoff oder Trifluormethyl,.
- R²¹: steht bevorzugt für Methyl, Ethyl oder C₁-C₂-Halogenalkyl mit 1 bis 5 Fluor, Chlor und/oder Bromatomen.
- R²¹: steht besonders bevorzugt für Methyl, Ethyl, Trifluormethyl, Difluormethyl, Difluorchlormethyl oder Trichlormethyl.
- R²¹: steht ganz besonders bevorzugt für Methyl, Trifluormethyl, Difluormethyl oder Trichlormethyl.
- R²²: steht bevorzugt für Methyl, Ethyl, Trifluormethyl, Difluormethyl, Difluorchlormethyl oder Trichlormethyl.
- R²²: steht besonders bevorzugt für Methyl, Trifluormethyl, Difluormethyl oder Trichlormethyl.
- R²³ und R²⁴: stehen unabhängig voneinander bevorzugt für Wasserstoff, Fluor, Chlor, Brom, Amino, Methyl, Ethyl oder C₁-C₂-Halogenalkyl mit 1 bis 5 Fluor, Chlor und/oder Bromatomen.
- R²³ und R²⁴: stehen unabhängig voneinander besonders bevorzugt für Wasserstoff, Fluor, Chlor, Brom, Methyl, Ethyl, Trifluormethyl, Difluormethyl, Difluorchlormethyl oder Trichlormethyl.
- R²³ und R²⁴: stehen unabhängig voneinander ganz besonders bevorzugt für Wasserstoff, Fluor, Chlor, Brom oder Methyl.
- R²³ und R²⁴: stehen insbesondere bevorzugt jeweils für Wasserstoff.
- R²⁵: steht bevorzugt für Wasserstoff, Methyl, Ethyl oder C₁-C₂-Halogenalkyl mit 1 bis 5 Fluor, Chlor und/oder Bromatomen.
- R²⁵: steht besonders bevorzugt für Wasserstoff, Methyl, Ethyl, Trifluormethyl, Difluormethyl, Difluorchlormethyl oder Trichlormethyl.
- R²⁵: steht ganz besonders bevorzugt für Wasserstoff, Methyl, Trifluormethyl, Difluormethyl oder Trichlormethyl.
- R²⁵: steht insbesondere bevorzugt für Methyl oder Trifluormethyl.
- R²⁶ und R²⁷: stehen unabhängig voneinander bevorzugt für Wasserstoff, Fluor, Chlor, Brom, Amino, Nitro, Methyl, Ethyl oder C₁-C₂-Halogenalkyl mit 1 bis 5 Fluor, Chlor und/oder Bromatomen.
- R²⁶ und R²⁷: stehen unabhängig voneinander besonders bevorzugt für Wasserstoff, Fluor, Chlor, Brom, Nitro, Methyl, Ethyl, Trifluormethyl, Difluormethyl, Difluorchlormethyl oder Trichlormethyl.
- R²⁶ und R²⁷: unabhängig voneinander ganz besonders bevorzugt für Wasserstoff, Fluor, Chlor, Brom oder Methyl.
- R²⁶ und R²⁷: stehen insbesondere bevorzugt jeweils für Wasserstoff.
- R²⁸: steht bevorzugt für Fluor, Chlor, Brom, Methyl, Ethyl oder C₁-C₂-Halogenalkyl mit 1 bis 5 Fluor, Chlor und/oder Bromatomen.
- R²⁸: steht besonders bevorzugt für Fluor, Chlor, Brom, Methyl, Ethyl, Trifluormethyl, Difluormethyl, Difluorchlormethyl oder Trichlormethyl.
- R²⁸: steht ganz besonders bevorzugt für Fluor, Chlor, Brom, Methyl, Trifluormethyl, Difluormethyl oder Trichlormethyl.
- R²⁸: steht insbesondere bevorzugt für Methyl.
- R²⁹: steht bevorzugt für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen.
- R²⁹: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R²⁹: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R²⁹: steht insbesondere bevorzugt für Wasserstoff, Chlor, Amino, Methylamino, Dimethylamino, Methyl oder Trifluormethyl.
- R³⁰: steht bevorzugt für Fluor, Chlor, Brom, Hydroxy, Methyl, Ethyl, Methoxy, Ethoxy, Cyclopropyl, C₁-C₂-Halogenalkyl oder C₁-C₂-Halogenalkoxy mit 1 bis 5 Fluor, Chlor und/oder Bromatomen.
- R³⁰: steht besonders bevorzugt für Fluor, Chlor, Brom, Hydroxy, Methyl, Ethyl, Methoxy, Ethoxy, Cyclopropyl, Trifluormethyl, Difluormethyl, Difluorchlormethyl oder Trichlormethyl.
- R³⁰: steht ganz besonders bevorzugt für Fluor, Chlor, Brom, Hydroxy, Methyl, Methoxy, Cyclopropyl, Trifluormethyl, Difluormethyl oder Trichlormethyl.
- R³¹: steht bevorzugt für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen.
- R³¹: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R³¹: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R³¹: steht insbesondere bevorzugt für Amino, Methylamino, Dimethylamino, Methyl oder Trifluormethyl.
- R³²: steht bevorzugt für Fluor, Chlor, Brom, Methyl, Ethyl oder C₁-C₂-Halogenalkyl mit 1 bis 5 Fluor, Chlor und/oder Bromatomen.
- R³²: steht besonders bevorzugt für Fluor, Chlor, Brom, Methyl, Ethyl, Trifluormethyl, Difluormethyl, Difluorchlormethyl oder Trichlormethyl.
- R³²: steht ganz besonders bevorzugt für Fluor, Chlor, Brom, Methyl, Trifluormethyl, Difluormethyl oder Trichlormethyl.
- R³²: steht insbesondere bevorzugt für Methyl, Trifluormethyl oder Difluormethyl.
- R³³: steht bevorzugt für Wasserstoff, Methyl oder Ethyl.
- R³³: steht besonders bevorzugt für Methyl.
- R³⁴: steht bevorzugt für Fluor, Chlor, Brom, Methyl oder Ethyl,
- R³⁴: steht besonders bevorzugt für Fluor, Chlor oder Methyl.
- R³⁵: steht bevorzugt für Methyl, Ethyl oder C₁-C₂-Halogenalkyl mit 1 bis 5 Fluor, Chlor und/oder Bromatomen.
- R³⁵: steht besonders bevorzugt für Methyl, Ethyl, Trifluormethyl, Difluormethyl, Difluorchlormethyl oder Trichlormethyl.
- R³⁵: steht ganz besonders bevorzugt für Methyl, Trifluormethyl, Difluormethyl oder Trichlormethyl.
- R³⁵: steht insbesondere bevorzugt für Methyl oder Trifluormethyl.
- R³⁶: steht bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R³⁶: steht besonders bevorzugt für Wasserstoff, Fluor, Chlor, Brom, Methyl oder Trifluormethyl.
- R³⁶: steht ganz besonders bevorzugt für Wasserstoff oder Chlor.
- R³⁷: steht bevorzugt für Fluor, Chlor, Brom, Iod, Hydroxy, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen.
- R³⁷: steht besonders bevorzugt für Fluor, Chlor, Brom, Iod, Methyl, Ethyl, n-Propyl, iso-Propyl, n-Butyl, iso-Butyl, sec-Butyl, tert-Butyl, Trifluormethyl, Difluormethyl, Difluorchlormethyl, Trichlormethyl.
- R³⁷: steht ganz besonders bevorzugt für Fluor, Chlor, Brom, Iod, Methyl, Trifluormethyl, Difluormethyl oder Trichlormethyl.
- R³⁸: steht bevorzugt für Wasserstoff, Methyl, Ethyl, C₁-C₂-Halogenalkyl mit 1 bis 5 Fluor-, Chlor- und/oder Bromatomen, C₁-C₂-Alkoxy-C₁-C₂-alkyl, Hydroxymethyl, Hydroxyethyl, methylsulfonyl oder Dimethylaminosulfonyl.
- R³⁸: steht besonders bevorzugt für Wasserstoff, Methyl, Ethyl, Trifluormethyl, Methoxymethyl, Ethoxymethyl, Hydroxymethyl oder Hydroxyethyl.
- R³⁸: steht ganz besonders bevorzugt für Methyl oder Methoxymethyl.
- R³⁹: steht bevorzugt für Wasserstoff, Fluor, Chlor, Brom, Methyl, Ethyl oder C₁-C₂-Halogenalkyl mit 1 bis 5 Fluor-, Chlor- und/oder Bromatomen.
- R³⁹: steht besonders bevorzugt für Wasserstoff, Fluor, Chlor, Brom, Methyl, Ethyl Trifluormethyl, Difluormethyl oder Trichlormethyl. 1.
- R³⁹: steht ganz besonders bevorzugt für Wasserstoff oder Methyl.
- R⁴⁰: steht bevorzugt für Wasserstoff, Fluor, Chlor, Brom, Cyano, Methyl, Ethyl, iso-Propyl oder C₁-C₂-Halogenalkyl mit 1 bis 5 Fluor-, Chlor- und/oder Bromatomen.
- R⁴⁰: steht besonders bevorzugt für Wasserstoff, Fluor, Chlor, Brom, Cyano, Methyl, Ethyl, iso-Propyl, Trifluormethyl, Difluormethyl, Difluorchlormethyl oder Trichlormethyl.
- R⁴⁰: steht ganz besonders bevorzugt für Wasserstoff, Fluor, Methyl oder Trifluormethyl.
- R⁴¹: steht bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R⁴¹: steht besonders bevorzugt für Wasserstoff, Fluor, Chlor, Brom, Iod, Methyl oder Trifluormethyl.
- R⁴¹: steht ganz besonders bevorzugt für Wasserstoff oder Trifluormethyl.
- R⁴²: steht bevorzugt für Methyl, Ethyl, n-Propyl oder iso-Propyl.
- R⁴²: steht besonders bevorzugt Methyl oder Ethyl.

Hervorgehoben sind Verbindungen der Formel (I), in welcher L für L-1 steht, wobei R² die oben angegebenen allgemeinen Bedeutungen hat.

Hervorgehoben sind Verbindungen der Formel (I), in welcher L für L-1 steht, wobei R² die oben angegebenen bevorzugten Bedeutungen hat.

Hervorgehoben sind Verbindungen der Formel (I), in welcher L für L-1 steht, wobei R² die oben angegebenen besonders bevorzugten Bedeutungen hat.

Hervorgehoben sind Verbindungen der Formel (I), in welcher L für L-1 steht, wobei R² die oben angegebenen ganz besonders bevorzugten Bedeutungen hat.

Hervorgehoben sind Verbindungen der Formel (I), in welcher L für L-1 steht, wobei R² die oben angegebenen insbesondere bevorzugten Bedeutungen hat.

Hervorgehoben sind Verbindungen der Formel (I), in welcher R¹ für Wasserstoff steht. Hervorgehoben sind Verbindungen der Formel (I), in welcher R¹ für Formyl steht.

Hervorgehoben sind außerdem Verbindungen der Formel (I), in welcher R¹ für -C(=O)C(=O)R⁴ steht, wobei R⁴ die oben angegebenen Bedeutungen hat.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Die genannten Definitionen können untereinander in beliebiger Weise kombiniert werden. Außerdem können auch einzelne Definitionen entfallen.

Bevorzugt, besonders bevorzugt oder ganz besonders bevorzugt sind Verbindungen der Formel (I), welche jeweils die unter bevorzugt, besonders bevorzugt oder ganz besonders bevorzugt genannten Substituenten tragen.

### Beschreibung der erfindungsgemäßen Verfahren zum Herstellen der Hexylcarboxanilide der Formel (I) sowie der Zwischenprodukte

### Verfahren (a)

Verwendet man 3-Dichlormethyl-1-methyl-1H-pyrazol-4-carbonyl-chlorid und [2-(1,3,3-Trimethylbutyl)phenyl]amin als Ausgangsstoffe, so kann das erfindungsgemäße Verfahren (a) durch das folgende Formelschema veranschaulicht werden:

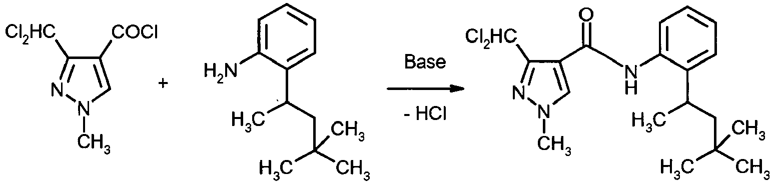

Die zur Durchführung des erfindungsgemäßen Verfahrens (a) als Ausgangsstoffe benötigten Carbonsäure-Derivate sind durch die Formel (II) allgemein definiert. In dieser Formel (II) hat A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für A angegeben wurden. X¹ steht bevorzugt für Chlor, Brom oder Hydroxy.

Die Carbonsäure-Derivate der Formel (II) sind größtenteils bekannt und/oder lassen sich nach bekannten Verfahren herstellen (vgl. WO 93/11117, EP-A 0 545 099, EP-A 0 589 301 und EP-A 0 589 313).
3-Dichlormethyl-1H-pyrazol-4-carbonsäure-Derivate der Formel (II-a) 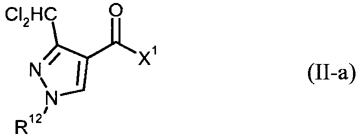 in welcher
- R¹²: die oben angegebenen Bedeutungen hat,
- X¹: für Halogen oder Hydroxy steht,
können erhalten werden, indem man in einem ersten Schritt Ketoacetale der Formel (V) 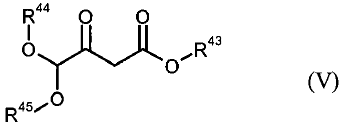 in welcher
R⁴³ für C₁-C₄-Alkyl, bevorzugt für Methyl, Ethyl, n-, iso-Propyl, n-, sec-, tert-Butyl, steht, R⁴⁴ und R⁴⁵ jeweils für Methyl oder Ethyl stehen, oder
R⁴⁴ und R⁴⁵ gemeinsam für -(CH₂)₃- oder -CH₂-C(CH₃)₂-CH₂- stehen,
mit Orthoameisensäurealkylestern der Formel (VI)

HC-(OR⁴⁶)₃ (VI)

in welcher
R⁴⁶ für C₁-C₄-Alkyl, bevorzugt für Methyl, Ethyl, n-, iso-Propyl, n-, sec-, tert-Butyl, steht, in Gegenwart eines Anhydrids (z.B. Essigsäureanhydrid) umsetzt
und die so erhaltenen Verbindungen der Formel (VII) 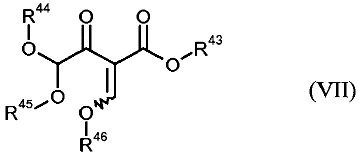 in welcher R⁴³, R⁴⁴, R⁴⁵ und R⁴⁶ die oben angegebenen Bedeutungen haben, in einem zweiten Schritt mit Hydrazin-Derivaten der Formel (VIII)

R¹²-NH-NH₂ (VIII)

in welcher R¹² die oben angegebenen Bedeutungen hat,
in Gegenwart eines Verdünnungsmittels (z.B. Methanol) umsetzt
und die so erhaltenen Pyrazol-Derivate der Formel (IX) 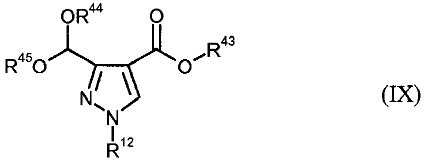 in welcher R¹², R⁴³, R⁴⁴ und R⁴⁵ die oben angegebenen Bedeutungen haben,
in einem dritten Schritt in Gegenwart einer Säure (z.B. Salzsäure) und in Gegenwart eines Verdünnungsmittels (z.B. Dioxan) umsetzt
und die so erhaltenen 3-Formyl-1H-pyrazol-4-carbonsäureester der Formel (X) 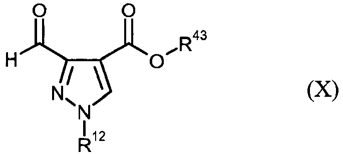 in welcher R¹² und R⁴³ die oben angegebenen Bedeutungen haben,
entweder
a) in einem vierten Schritt in Gegenwart einer Base (z.B. Lithiumhydroxid) und in Gegenwart eines Verdünnungsmittels (z.B. Tetrahydrofuran) verseift
   und die so erhaltenen 3-Formyl-1H-pyrazol-4-carbonsäuren der Formel (XI) 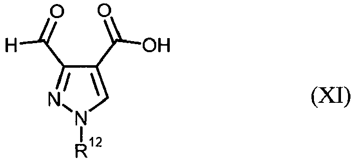 in welcher R¹² die oben angegebenen Bedeutungen hat,
   anschließend mit einem Chlorierungsmittel (z.B. Phosphorpentachlorid) in Gegenwart eines Verdünnungsmittels (z.B. Dichlormethan) umsetzt,
   oder
b) in einem vierten Schritt mit einem Chlorierungsmittel (z.B. Phosphorpentachlorid) in Gegenwart eines Verdünnungsmittels (z.B. Dichlormethan) umsetzt
   und die so erhaltenen 3-Dichlormethyl-1H-pyrazol-4-carbonsäureester der Formel (XII) 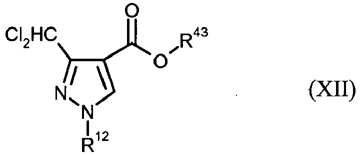 in welcher R¹² und R⁴³ die oben angegebenen Bedeutungen haben,
   anschließend in Gegenwart einer Base (z.B. Lithiumhydroxid) und in Gegenwart eines Verdünnungsmittels (z.B. Tetrahydrofuran) verseift.

Die zur Durchführung des erfindungsgemäßen Verfahrens (a) als Ausgangsstoffe weiterhin benötigten Anilin-Derivate sind durch die Formel (III) allgemein definiert. In dieser Formel (III) haben L, R¹ und R³ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für diese Reste als bevorzugt, besonders bevorzugt bzw. ganz besonders bevorzugt angegeben wurden.

Die Anilin-Derivate der Formel (III), in denen L für L-1 steht, sind teilweise neu. Anilin-Derivate der Formel (III), in denen L für L-1 steht, lassen sich herstellen, indem man
c) in einem ersten Schritt ein Anilin-Derivat der Formel (XIII) 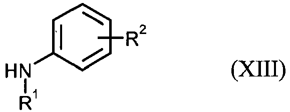 in welcher R¹ und R² die oben angegebenen Bedeutungen haben,
   mit einem Alken der Formel (XIV) 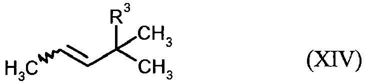 in welcher R³ die oben angegebenen Bedeutungen hat,
   in Gegenwart eines Katalysators, gegebenenfalls in Gegenwart einer Base und gegebenenfalls in Gegenwart eines Verdünnungsmittels umsetzt,
   und das so erhaltene Alkenanilin der Formel (XV) 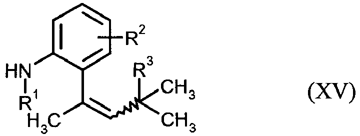 in welcher R¹, R² und R³ die oben angegebenen Bedeutungen haben,
   in einem zweiten Schritt gegebenenfalls in Gegenwart eines Verdünnungsmittels und gegebenenfalls in Gegenwart eines Katalysators hydriert.

Die zur Durchführung des erfindungsgemäßen Verfahrens (c) als Ausgangsstoffe benötigten Anilin-Derivate sind durch die Formel (XIII) allgemein definiert. In dieser Formel (XIII) haben R¹ und R² bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für diese Reste als bevorzugt, besonders bevorzugt bzw. ganz besonders bevorzugt angegeben wurden.

Anilin-Derivate der Formel (XIII) sind bekannt oder können nach bekannten Methoden erhalten werden. Anilin-Derivate der Formel (XIII), in welcher R¹ nicht für Wasserstoff steht, können erhalten werden, indem man Aniline der Formel (XIII-a) 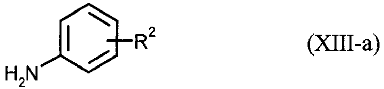 in welcher R² die oben angegebenen Bedeutungen hat,
mit Halogeniden der Formel (IV)

R^{1-A}-X² (IV)

in welcher R^{1-A} und X² die oben angegebenen Bedeutungen hat,
in Gegenwart einer Base und in Gegenwart eines Verdünnungsmittels umsetzt. [Die Reaktionsbedingungen des Verfahrens (b) gelten entsprechend.]

Die zur Durchführung des erfindungsgemäßen Verfahrens (c) als Ausgangsstoffe weiterhin benötigten Alkene sind durch die Formel (XIV) allgemein definiert. In dieser Formel (XIV) hat R³ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für diesen Rest als bevorzugt, besonders bevorzugt bzw. ganz besonders bevorzugt angegeben wurden.

Alkene der Formel (XTV) sind bekannt oder können nach bekannten Methoden erhalten werden.

Die bei der Durchführung des erfindungsgemäßen Verfahrens (c) als Zwischenprodukte durchlaufenen Alkenaniline sind durch die Formel (XV) allgemein definiert. In dieser Formel (XV) haben R¹, R² und R³ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für diese Reste als bevorzugt, besonders bevorzugt bzw. ganz besonders bevorzugt angegeben wurden.

Alkenaniline der Formel (XV) sind teilweise bekannt.

Das erfindungsgemäße Verfahren (c) kann in verschiedenen Varianten durchgeführt werden. So ist es möglich, zunächst Aniline der Formel (XIII-a) mit Alkenen der Formel (XIV) zu den entsprechenden Anilin-Derivaten der Formel (III-a) 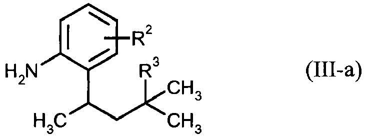 in welcher R² und R³ die oben angegebenen Bedeutungen haben, umzusetzen, welche dann gegebenenfalls mit Halogeniden der Formel (IV)

R^{1-A}-X² (IV)

in welcher R^{1-A} und X² die oben angegebenen Bedeutungen haben,
in Gegenwart einer Base und in Gegenwart eines Verdünnungsmittels zu den entsprechenden Anilin-Derivaten der Formel (III) umgesetzt werden. [Die Reaktionsbedingungen des Verfahrens (b) gelten entsprechend.]

Es ist jedoch auch möglich, auf der Stufe der Alkenaniline der Formel (XV) die Umsetzung mit einem Halogenid der Formel (IV) durchzuführen und anschließen zu hydrieren.
Anilin-Derivate der Formel (III-b) 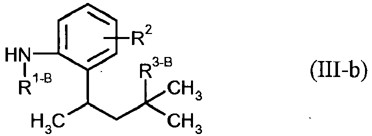 in welcher
a) R^{1-B} für Wasserstoff steht, und
   R^{3-B} für Halogen, C₃-C₈-Alkyl, C₁-C₈-Halogenalkyl steht,
   oder
b) R^{1-B} für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)-carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
   (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogen-alkoxy)carbonyl, (Halogen-C₁-C₄-alkoxy-C₁-C₄-alkyl)carbonyl, (C₃-C₈-Halogen-cycloalkyl)carbonyl mit jeweils 1 bis 9 Fluor-, Chlor- und/oder Bromatomen; oder -C(=O)C(=O)R⁴, -CONR⁵R⁶ oder -CH₂NR⁷R⁸ steht, und
   R^{3-B} für Wasserstoff, Halogen, C₁-C₈-Alkyl, C₁-C₈-Halogenalkyl steht,
und
R², R⁴, R⁵, R⁶, R⁷ und R⁸ jeweils die oben angegebenen Bedeutungen haben, sind neu und ebenfalls Gegenstand dieser Anmeldung.

Die bevorzugten, besonders bevorzugten bzw. ganz besonders bevorzugten Bedeutungen von R¹ und R³ finden auf R^{1-B} und R^{3-B} entsprechend Anwendung, wobei im Fall a) R^{1-B} immer für Wasserstoff und R^{3-B} nicht für Wasserstoff, Methyl oder Ethyl steht und im Fall b) R^{1-B} nicht für Wasserstoff steht. Die bevorzugten, besonders bevorzugten bzw. ganz besonders bevorzugten Bedeutungen von R², R⁴, R⁵, R⁶, R⁷ und R⁸ gelten ebenfalls für die neuen Verbindungen der Formel (III-b).

Hervorgehoben sind Verbindungen der Formel (III-b), in welcher R¹ und R² jeweils für Wasserstoff und R³ für Fluor, Chlor, Methyl, Ethyl, Trifluormethyl oder Pentafluorethyl steht.

Die Anilin-Derivate der Formel (III), in denen L für L-2, L-3 oder L-4 steht, sind bekannt und/oder können nach bekannten Verfahren erhalten werden (vgl. z.B. EP-A 1 036 793 und EP-A 0 737 682).

Anilin-Derivate der Formel (III), in denen L für L-2, L-3 oder L-4 steht und R¹ nicht für Wasserstoff steht, können erhalten werden, indem man Aniline der Formel (III-c) 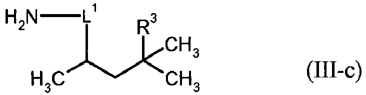 in welcher
L¹ für L-2, L-3 oder L-4 steht und
L-2, L-3, L-4 und R³ die oben angegebenen Bedeutungen haben,
mit Halogeniden der Formel (IV)

R^{1-A}-X² (IV)

in welcher R^{1-A} und X² die oben angegebenen Bedeutungen hat,
in Gegenwart einer Base und in Gegenwart eines Verdünnungsmittels umsetzt. [Die Reaktionsbedingungen des Verfahrens (b) gelten entsprechend.]

### Verfahren (b)

Verwendet man 1,3,5-Trimethyl-N-[2-(1,3,3-trimethylbutyl)phenyl]-1H-pyrazol-4-carboxamid und Ethyl-chlor(oxo)acetat als Ausgangsstoffe, so kann der Verlauf des erfindungsgemäßen Verfahrens (b) durch das folgende Formelschema veranschaulicht werden:

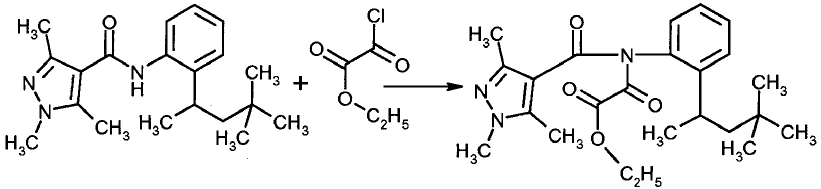

Die zur Durchführung des erfindungsgemäßen Verfahrens (b) als Ausgangsstoffe benötigten Hexylcarboxanilide sind durch die Formel (I-a) allgemein definiert. In dieser Formel (I-a) haben R², R³ und A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Hexylcarboxanilide der Formel (I-a) sind ebenfalls erfindungsgemäße Verbindungen und Gegenstand dieser Anmeldung. Sie können nach dem erfindungsgemäßen Verfahren (a) erhalten werden (mit R¹ = Wasserstoff).

Die zur Durchführung des erfindungsgemäßen Verfahrens (b) als Ausgangsstoffe weiterhin benötigten Halogenide sind durch die Formel (IV) allgemein definiert.
- R^{1-A}: steht bevorzugt für C₁-C₆-Alkyl, C₁-C₄-Alkylsulfinyl, C₁-C₄-Aklsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₆- Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R⁴, -CONR⁵R⁶ oder -CH₂NR⁷R⁸.
- R^{1-A}: steht besonders bevorzugt für Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl; Formyl, -CH₂-CHO, -(CH₂)₂-CHO, -CH₂-CO-CH₃, -CH₂-CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂-CO₂CH₃, -CH₂-CO₂CH₂CH₃, -CH₂-CO₂CH(CH₃)₂, -(CH₂)₂-CO₂CH₃, -(CH₂)₂-CO₂CH₂CH₃, -(CH₂)₂-CO₂CH(CH₃)₂, -CH₂-CO-CF₃, -CH₂-CO-CCl₃, -CH₂-CO-CH₂CF₃, -CH₂-CO-CH₂CCl₃, -(CH₂)₂-CO-CH₂CF₃, -(CH₂)₂-CO-CH₂CCl₃, -CH₂-CO₂CH₂CF₃, -CH₂-CO₂CF₂CF₃, -CH₂-CO₂CH₂CCl₃, -CH₂-CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CCl₃;
Methylcarbonyl, Ethylcarbonyl, n-Propylcarbonyl, iso-Propylcarbonyl, tert-Butylcarbonyl, Methoxycarbonyl, Ethoxycarbonyl, tert-Butoxycarbonyl, Cyclopropylcarbonyl; Trifluormethylcarbonyl, Trifluormethoxycarbonyl, oder -C(=O)C(=O)R⁵, -CONR⁶R⁷oder -CH₂NR⁸R⁹.
- R^{1-A}: steht ganz besonders bevorzugt für Methyl, Methoxymethyl, Formyl, -CH₂-CHO, -(CH₂)₂-CHO, -CH₂-CO-CH₃, -CH₂-CO-CH₂CH₃, -CH₂-CO-CH(CH₃)₂, -C(=O)CHO, -C(=O)C(=O)CH₃, -C(=O)C(=O)CH₂OCH₃, -C(=O)CO₂CH₃, -C(=O)CO₂CH₂CH₃.
- X²: steht bevorzugt für Chlor oder Brom.

Halogenide der Formel (IV) sind bekannt.

### Reaktionsbedingungen

Als Verdünnungsmittel zur Durchführung des erfindungsgemäßen Verfahrens (a) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (a) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (a) wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ), Triphenylphosphin/Tetrachlorkohlenstoff oder Brom-tripyrrolidinophosphonium-hexafluorophosphat.

Das erfindungsgemäße Verfahren (a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens (a) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens (a) zur Herstellung der Verbindungen der Formel (I) setzt man pro mol des Carbonsäure-Derivates der Formel (II) im Allgemeinen 0,2 bis 5 mol, vorzugsweise 0,5 bis 2 mol an Anilin-Derivat der Formel (III) ein.

Als Verdünnungsmittel zur Durchführung des erfindungsgemäßen Verfahrens (b) kommen alle inerten organischen Lösungsmittel in Betracht Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (b) wird in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).
Die Reaktionstemperaturen können bei der Durchführung des erfindungsgemäßen Verfahrens (b) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens (b) zur Herstellung der Verbindungen der Formel (I) setzt man pro Mol des Hexylcarboxanilids der Formel (I-a) im Allgemeinen 0,2 bis 5 Mol, vorzugsweise 0,5 bis 2 Mol an Halogenid der Formel (IV) ein.

Als Verdünnungsmittel zur Durchführung des ersten Schrittes des erfindungsgemäßen Verfahrens (c) kommen alle inerten organischen Lösungsmittel in Betracht. Hierzu gehören vorzugsweise Nitrile, wie Acetonitril, Propionitril, n- oder i-Butyronitril oder Benzonitril oder Amide, wie N,N-Dimethylformamid, N,N-Dimethylacetamid, N-Methylformanilid, N-Methylpyrrolidon oder Hexamethylphosphorsäuretriamid.

Der erste Schritt des erfindungsgemäßen Verfahrens (c) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methyhnorpholin, N,N-Dimethylaminopyridin, Diazabicyclooctan (DABCO), Diazabicyclononen (DBN) oder Diazabicycloundecen (DBU).

Der erste Schritt des erfindungsgemäßen Verfahrens (c) wird in Gegenwart eines oder mehrerer Katalysatoren durchgeführt.

Dazu eignen sich besonders Palladiumsalze oder -komplexe. Hierzu kommen vorzugsweise Palladiumchlorid, Palladiumacetat, Tetrakis-(triphenylphosphin)-Palladium oder Bis-(triphenylphosphin)-Palladiumdichlorid infrage. Es kann auch ein Palladiumkomplex in der Reaktionsmischung erzeugt werden, wenn man ein Palladiumsalz und ein Komplexligand getrennt zur Reaktion zugibt.

Als Liganden kommen vorzugsweise Organophosphorverbindungen infrage. Beispielhaft seien genannt: Triphenylphosphin, tri-o-Tolylphosphin, 2,2'-Bis(diphenylphosphino)-1,1'-binaphthyl, Dicyclohexylphosphinebiphenyl, 1,4-Bis(diphenylphosphino)butan, Bisdiphenylphosphinoferrocen, Di(tert.-butylphosphino)biphenyl, Di(cyclohexylphosphino)biphenyl, 2-Dicyclohexylphosphino-2`-N,N-dimethylaminobiphenyl, Tricyclohexylphosphin, Tri-tert.-butylphosphin. Es kann aber auch auf Liganden verzichtet werden.

Der erste Schritt des erfindungsgemäßen Verfahrens (c) wird ferner gegebenenfalls in Gegenwart eines weiteren Metallsalzes, wie Kupfersalzen, beispielsweise Kupfer-(I)-iodid durchgeführt.

Die Reaktionstemperaturen können bei der Durchführung des ersten Schrittes des erfindungsgemäßen Verfahrens (c) in einem größeren Bereich variiert werden. Im allgemeinen arbeitet man bei Temperaturen von 20°C bis 180°C, vorzugsweise bei Temperaturen von 50°C bis 150°C.

Zur Durchführung des ersten Schrittes des erfindungsgemäßen Verfahrens (c) zur Herstellung der Alkenaniline der Formel (XV) setzt man pro Mol des Anilin-Derivates der Formel (XIII) im Allgemeinen 1 bis 5 mol, vorzugsweise 1 bis 3 mol an Alken der Formel (XIV) ein.

Als Verdünnungsmittel zur Durchführung des zweiten Schrittes (Hydrierung) des erfindungsgemäßen Verfahrens (c) kommen alle inerten organischen Lösungsmittel in Betracht. Hierzu gehören vorzugsweise aliphatische oder alicyclische Kohlenwasserstoffe, wie z.B. Petrolether, Hexan, Heptan, Cyclohexan, Methylcyclohexan oder Decalin; Ether, wie Diethylether, Diisopropylether, Methyl-tert-butylether, Methyl-tert-amylether, Dioxan, Tetrahydrofuran, 1,2- Dimethoxyethan oder 1,2-Diethoxyethan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Der zweite Schritt (Hydrierung) des erfindungsgemäßen Verfahrens (c) wird in Gegenwart eines Katalysators durchgeführt. Als solche kommen alle Katalysatoren infrage, die für Hydrierungen üblicherweise verwendet werden. Beispielhaft seien genannt: Raney-Nickel, Palladium oder Platin, gegebenenfalls auf einem Trägermaterial, wie z.B. Aktivkohle.
Die Hydrierung im zweiten Schritt des erfindungsgemäßen Verfahrens (c) kann statt in Gegenwart von Wasserstoff in Kombination mit einem Katalysator auch in Anwesenheit von Triethylsilan durchgeführt werden.

Die Reaktionstemperaturen können bei der Durchführung des zweiten Schrittes des erfindungsgemäßen Verfahrens (c) in einem größeren Bereich variiert werden. Im Allgemeinen arbeitet man bei Temperaturen von 0°C bis 150°C, vorzugsweise bei Temperaturen von 20°C bis 100°C.

Der zweite Schritt des erfindungsgemäßen Verfahrens (c) wird unter einem Wasserstoffdruck zwischen 0.5 and 200 bar, bevorzugt zwischen 2 und 50 bar, besonders bevorzugt zwischen 3 und 10 bar durchgeführt.

Wenn nicht anders angegeben, werden alle erfindungsgemäßen Verfahren im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie z.B. Xanthomonas campestris pv. oryzae;
Pseudomonas-Arten, wie z.B. Pseudomonas syringae pv. lachrymans;
Erwinia-Arten, wie z.B. Erwinia amylovora;
Pythium-Arten, wie z.B. Pythium ultimum;
Phytophthora-Arten, wie z.B. Phytophthora infestans;
Pseudoperonospora-Arten, wie z.B. Pseudoperonospora humuli oder
Pseudoperonospora cubensis;
Plasmopara-Arten, wie z.B. Plasmopara viticola;
Bremia-Arten, wie z.B. Bremia lactucae;
Peronospora-Arten, wie z.B. Peronospora pisi oder P. brassicae;
Erysiphe-Arten, wie z.B. Erysiphe graminis;
Sphaerotheca-Arten, wie z.B. Sphaerotheca fuliginea;
Podosphaera-Arten, wie z.B. Podosphaera leucotricha;
Venturia-Arten, wie z.B. Venturia inaequalis;
Pyrenophora-Arten, wie z.B. Pyrenophora teres oder P. graminea
(Konidienform: Drechslera, Syn: Helminthosporium);
Cochliobolus-Arten, wie z.B. Cochliobolus sativus
(Konidienform: Drechslera, Syn: Helminthosporium);
Uromyces-Arten, wie z.B. Uromyces appendiculatus;
Puccinia-Arten, wie z.B. Puccinia recondita;
Sclerotinia-Arten, wie z.B. Sclerotinia sclerotiorum;
Tilletia-Arten, wie z.B. Tilletia caries;
Ustilago-Arten, wie z.B. Ustilago nuda oder Ustilago avenae;
Pellicularia-Arten, wie z.B. Pellicularia sasakii;
Pyricularia-Arten, wie z.B. Pyricularia oryzae;
Fusarium-Arten, wie z.B. Fusarium culmorum;
Botrytis-Arten, wie z.B. Botrytis cinerea;
Septoria-Arten, wie z.B. Septoria nodorum;
Leptosphaeria-Arten, wie z.B. Leptosphaeria nodorum;
Cercospora-Arten, wie z.B. Cercospora canescens;
Alternaria-Arten, wie z.B. Alternaria brassicae;
Pseudocercosporella-Arten, wie z.B. Pseudocercosporella herpotrichoides,
Rhizoetonia-Arten, wie z.B. Rhizoctonia solani.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globösum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.
Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen 'anfrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfat, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.
Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:
**Fungizide:**
   2-Phenylphenol; 8-Hydroxychinolinsulfat; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamin; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover, Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr,, Furcarbanil; Furmacyclox; Guazatine; Hexachlorobenzene; Hexaconazole; Hymexazol; Imazalil; Imibenconazole; Iminoctadine triacetate; Iminoctadine tris(albesil; Iodo- carb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2-propinyl]oxy]-3-methoxyphenyl]ethyl]-3-methyl-2-[(methylsulfonyl)amino]-butanamid; 1-(1-Naphthalenyl)-1H-pyrrol-2,5-dion; 2,3,5,6-Tetrachlor-4-(methylsulfonyl)pyridin; 2-Amino-4-methyl-N-phenyl-5-thiazolcarboxamid; 2-Chlor-N-(2,3-dihydro-1,1,3-trimethyl-1H-inden-4-yl)-3-pyridincarboxamide; 3,4,5-Trichlor-2,6-pyridindicarbonitril; Actinovate; cis-1-(4-Chlorphenyl)-2-(1H-1,2,4-triazol-1-yl)-cycloheptanol; Methyl 1-(2,3-dihydro-2,2-dimethyl-1H-inden-1-yl)-1H-imidazol-5-carboxylat; Monokaliumcarbonat; N-(6-Methoxy-3-pyridinyl)-cyclopropan-carboxamid; N-Butyl-8-(1,1-dimethylethyl)-1-oxaspiro[4.5]decan-3-amin; Natriumtetrathiocarbonat; sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper; Oxine-copper.
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin, Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Benclothiaz, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben, Cadusafos, Camphechlor, Carbaryl, Carbofuran, Carbophenothion, Carbosulfan, Cartap, CGA-50439, Chinomethionat, Chlordane, Chlordimefoim, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine, DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimefluthrin, Dimethoate, Dimethylvinphos, Dinobuton, Dinocap, Dinotefuran, Diofenolan, Disulfoton, Docusat-sodium, Dofenapyn, DOWCO-439, Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos, Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb, Gamma-Cyhalothrin, Gamma-HCH, Gossyplure, Grandlure, Granuloseviren, Halfenprox, Halofenozide, HCH, HCN-801, Heptenophos, Hexaflumuron, Hexythiazox, Hydramethylnone, Hydroprene, IKA-2002, Imidacloprid, Imiprothrin, Indoxacarb, Iodofenphos, Iprobenfos, Isazofos, Isofenphos, Isoprocarb, Isoxathion, Ivermectin, Japonilure, Kadethrin, Kernpolyederviren, Kinoprene, Lambda-Cyhalothrin, Lindane, Lufenuron, Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metofluthrin, Metolcarb, Metoxadiazone, Mevinphos, Milbemectin, Milbemycin, MKI-245, MON-45700, Monocrotophos, Moxidectin, MTI-800, Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron, OK-5101, OK-5201, OK-9601, OK-9602, OK-9701, OK-9802, Omethoate, Oxamyl, Oxydemeton-methyl, Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphosethyl, Potassium oleate, Prallethrin, Profenofos, Profluthrin, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen, Quinalphos, Resmethrin, RH-5849, Ribavirin, RU-12457, RU-15525, S-421, S-1833, Salithion, Sebufos, SI-0009, Silafluofen, Spinosad, Spirodiclofen, Spiromesifen, Sulfluramid, Sulfotep, Sulprofos, SZI-121, Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (IR-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Trichoderma atroviride, Triflumuron, Trimethacarb, Vamidothion, Vaniliprole, Verbutin, Verticillium lecanü, WL-108477, WL-40027, YI-5201, YI-5301, YI-5302, XMC, Xylylcarb, ZA-3274, Zeta-Cypermethrin, Zolaprofos, ZXI-8901, die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im Allgemeinen zwischen 0,1 und 10.000 g/ha, vorzugsweise zwischen 10 und 1.000 g/ha. Bei der Saatgutbehandlung liegen die Aufwandmengen an Wirkstoff im Allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleimt. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und Cry1F sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Zink® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

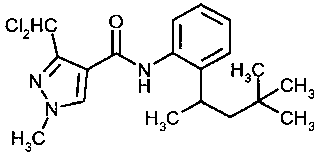

Zu einer Lösung bestehend aus 250.2 mg (1.1 mmol) 3-Dichlormethyl-1-methyl-1H-pyrazol-4-carbonylchlorid und 161.9 mg (1.6 mmol) Triethylamin in 10 ml Tetrahydrofuran werden 191.3 mg (1.0 mmol) [2-(1,3,3-Trimethylbutyl)phenyl]amin gegeben. Die Reaktionslösung wird 16 h bei 60°C gerührt, über Silica filtriert und im Vakuum aufkonzentriert.

Säulenchromatographie (Cyclohexan/Essigsäureethylester 3:1) liefert 342.1 mg (89 % der Theorie) an 3-(Dichlormethyl)-1-methyl-N-[2-(1,3,3-trimethylbutyl)phenyl]-1H-pyrazol-4-carboxamid [logP (pH = 2.3) = 4.02].

Analog Beispiel 1, sowie entsprechend den Angaben in den allgemeinen Verfahrensbeschreibungen, werden die in der nachstehenden Tabelle 1 genannten Verbindungen der Formel (I) erhalten.

**Tabelle 1**

| 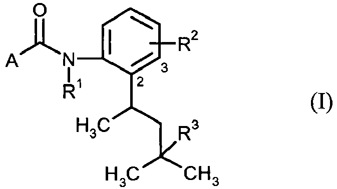 | | | | | | | | | | | | |
|---|---|---|---|---|---|---|---|---|---|---|---|---|
| **Bsp.** | **R¹** | **R²** | **R³** | **A** | **logP** | | **Bsp.** | **R¹** | **R²** | **R³** | **A** | **logP** |
| 2 | H | H | CH₃ | 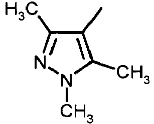 | 3.19 | | 3 | H | H | CH₃ | 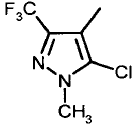 | 4.34 |
| 4 | H | H | CH₃ | 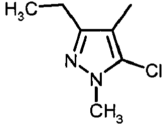 | 4.25 | | 5 | H | H | CH₃ | 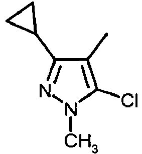 | 4.39 |
| 6 | H | H | CH₃ | 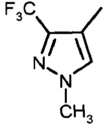 | 3.81 | | 7 | H | H | C₂H₅ | 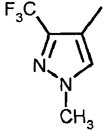 | 4.13 |
| 8 | H | H | CH₃ | 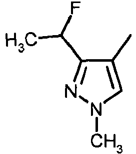 | 3.63 | | 9 | H | H | CH₃ | 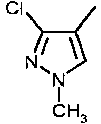 | 3.79 |
| 10 | H | H | CH₃ | 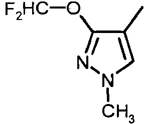 | 4.19 | | 11 | H | H | CH₃ | 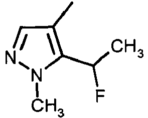 | 3.81 |
| 12 | H | H | CH₃ | 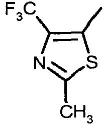 | 4.24 | | 13 | H | H | CH₃ | 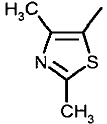 | 3.60 |
| 14 | H | H | CH₃ | 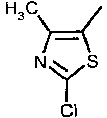 | 4.52 | | 15 | H | H | C₂H₅ | 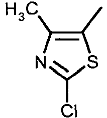 | 4.89 |
| 16 | H | H | CH₃ | 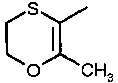 | 4.27 | | 17 | H | H | C₂H₅ | 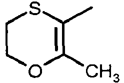 | 4.63 |
| 18 | H | H | CH₃ | 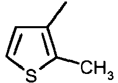 | 4.39 | | 19 | H | H | CH₃ | 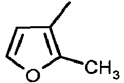 | 4.04 |
| 20 | H | H | C₂H₅ | 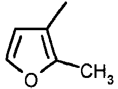 | 4.38 | | 21 | H | H | CH₃ | 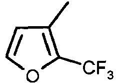 | 4.37 |
| 22 | H | H | CH₃ | 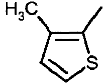 | 4.40 | | 23 | H | H | C₂H₅ | 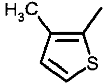 | 4.75 |
| 24 | H | H | CH₃ | 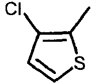 | 4.92 | | 25 | H | H | CH₃ | 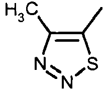 | 3.84 |
| 26 | H | H | CH₃ | 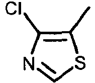 | 4.15 | | 27 | H | H | CH₃ | 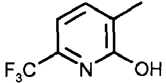 | 3.97 |
| 28 | H | H | CH₃ | 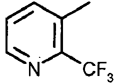 | 3.89 | | 29 | H | H | CH₃ | 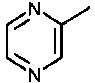 | 3.97 |
| 30 | H | H | CH₃ | 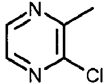 | 3.95 | | 31 | H | H | CH₃ | 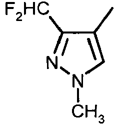 | 4.16 |
| 32 | H | H | CH₃ | 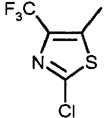 | 4.80 | | | | | | | |

### Herstellung von Ausgangsstoffen der Formel (II)

### Beispiel (II-1)

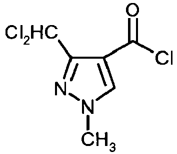

300.0 mg (1.9 mmol) 3-Formyl-1-methyl-1H-pyrazole-4-carbonsäure werden in 60 ml Dichlormethan gelöst und mit 1.0 g (4.9 mmol) Phosphorpentachlorid versetzt. Nach 1.5 h bei Raumtemperatur wird auf Eiswasser gegeben, mit Dichlormethan extrahiert, über Magnesiumsulfat getrocknet, filtriert und im Vakuum aufkonzentriert.
Man erhält so 384.0 mg (86 % der Theorie) an 3-Dichlormethyl-1-methyl-1H-pyrazol-4-carbonylchlorid [logP (pH 2.3) = 1.80].

### Herstellung von Ausgangsstoffen der Formel (VII)

### Beispiel (VII-1)

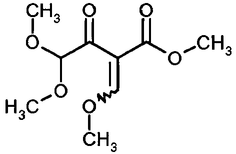

Zu einer Lösung bestehend aus 10.0 g (57 mmol) 4,4-Dimethoxy-3-oxo-buttersäure-methylester in 9.0 g (85 mmol) Orthoameisensäuretrimethylester werden 16.0 ml (170 mol) Essigsäureanhydrid gegeben. Die Reaktionsmischung wird für 16 h unter Rückfluss erhitzt.
Destillation aus der Reaktionsmischung (Siedepunkt 132-135°C, 0.2 bar) liefert 7.0 g (56 % der Theorie) an 4,4-Dimethoxy-2-methoxymethylen-3-oxo-buttersäuremethylester.

### Herstellung von Ausgangsstoffen der Formel (IX)

### Beispiel (IX-1)

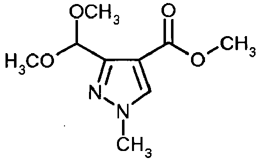

Bei -5°C wird eine Lösung bestehend aus 2.0 ml (38 mmol) Methylhydrazin in 340 ml Methanol langsam zu 7.5 g 4,4-Dimethoxy-2-methoxymethylen-3-oxo-buttersäuremethylester getropft. Nach beendeter Zugabe wird die Reaktionsmischung für 16 h bei Raumtemperatur gerührt und im Vakuum aufkonzentriert.
Säulenchromatographie (Laufmittelgradient Cyclohexan/Essigsäureethylester) liefert 6.5 g (77 % der Theorie) an 3-Dimethoxymethyl-1-methyl-1H-pyrazol-4-carbonsäuremethylester.

### Herstellung von Ausgangsstoffen der Formel (X)

### Beispiel (X-1)

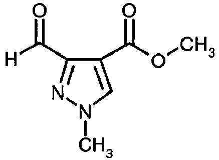

Eine Lösung aus 2.1 g (10 mmol) 3-Dimethoxymethyl-1-methyl-1H-pyrazol-4-carbonsäuremethylester in 20 ml Dioxan wird mit 10 ml konzentrierter Salzsäure versetzt und für 16 h bei Raumtemperatur gerührt. Zur Aufarbeitung wird im Vakuum aufkonzentriert, der Rückstand mit 200 ml Methylenchlorid aufgenommen und mit 50 ml Wasser gewaschen. Die organische Phase wird über Magnesiumsulfat getrocknet, filtriert und aufkonzentriert.
Man erhält 1.6 g (94 % der Theorie) an 3-Formyl-1-methyl-1H-pyrazole-4-carbonsäuremethylester [logP (pH 2.3) = 0.46].

### Herstellung von Ausgangsstoffen der Formel (XI)

### Beispiel (XI-1)

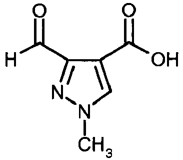

6.0 g (35.68 mmol) 3-Formyl-1-methyl-1H-pyrazol-4-carbonsäuremethylester werden in 180 ml Tetrahydrofuran und 90 ml Wasser gelöst und mit 0.94 g (39.25 mmol) Lithiumhydroxid versetzt. Die Reaktionsmischung wird für 16 h bei Raumtemperatur gerührt, das organische Lösungsmittel im Vakuum entfernt, die verbleibende wässrige Phase mit verdünnter Salzsäure angesäuert, dreimal mit je 100 ml Essigsäureethylester extrahiert. Die organischen Phasen werden über Magnesiumsulfat getrocknet, filtriert und aufkonzentriert.
Man erhält so 4.28 g (78 % der Theorie) an 3-Formyl-1-methyl-1H-pyrazole-4-carbonsäure mit dem logP (pH = 2.3) = -0.19.

### Herstellung von Ausgangsstoffen der Formel (XII)

### Beispiel (XII-1)

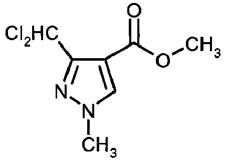

46.1 mg (0.27 mmol) 3-Formyl-1-methyl-1H-pyrazol-4-carbonsäuremethylester werden in 10 ml Dichlormethan gelöst und mit 142.9 mg (0.67 mmol) Phosphorpentachlorid versetzt. Die Reaktionsmischung wird für 1.5 h bei Raumtemperatur gerührt, auf Wasser gegeben, mit Diethylether extrahiert, über Magnesiumsulfat getrocknet und im Vakuum konzentriert.
Man erhält so 53.0 mg (86 % der Theorie) an 3-(Dichlormethyl)-1-methyl-1H-pyrazol-4-carbonsäuremethylester mit dem logP (pH 2.3) = 1.80.

Dieser Methylester kann auf übliche Weise verseift werden. Man erhält die 3-(Dichlormethyl)-1-methyl-1H-pyrazol-4-carbonsäure, welche entweder direkt mit Verbindungen der Formel (III) gekuppelt wird oder zuvor in das Säurechlorid überführt wird.

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.
Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.
Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).
Die lambda-max-Werte wurden an Hand der UV-Spektren von 200 nm bis 400 nm in den Maxima der chromatographischen Signale ermittelt.

### Anwendungsbeispiele

(Wirkstoffe, welche nicht unter Formel (I) der Ansprüche fallen, sind nicht erfindungsgemäss).

### Beispiel A

**Podosphaera-Test (Apfel) / protektiv**

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Podosphaera-Test (Apfel) / protektiv** | | |
|---|---|---|
| Wirkstoff | Aufwandmenge an Wirkstoff in g/ha | Wirkungsgrad in % |
| 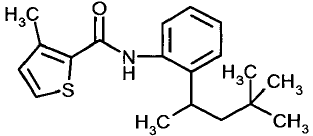 | 100 | 85 |
| 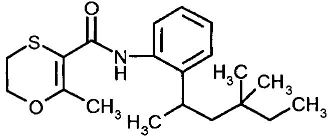 | 100 | 94 |
| 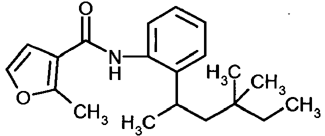 | 100 | 91 |
| 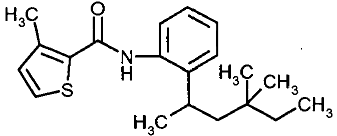 | 100 | 98 |
| 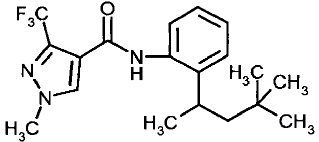 | 100 | 95 |
| 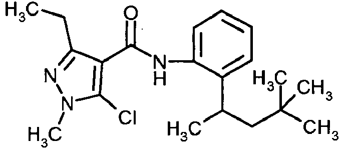 | 100 | 96 |
| 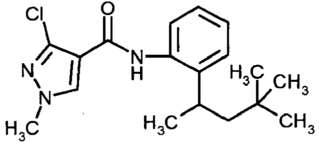 | 100 | 100 |
| 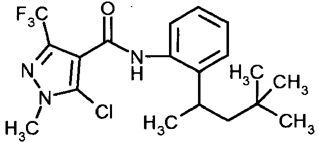 | 100 | 97 |
| 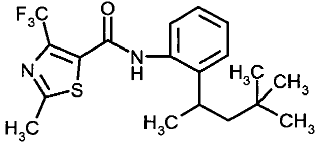 | 100 | 100 |

### Beispiel B

**Venturia - Test (Apfel) / protektiv**

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Venturia - Test (Apfel) / protektiv** | | |
|---|---|---|
| Wirkstoff | Aufwandmenge an Wirkstoff in g/ha | Wirkungsgrad in % |
| 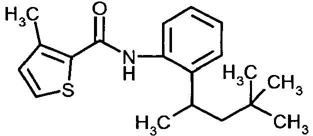 | 100 | 96 |
| 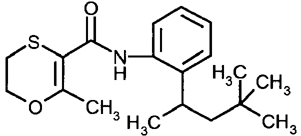 | 100 | 100 |
| 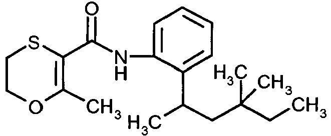 | 100 | 100 |
| 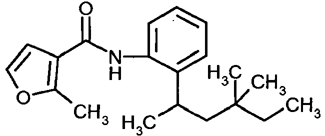 | 100 | 93 |
| 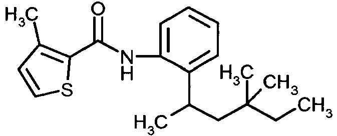 | 100 | 99 |
| 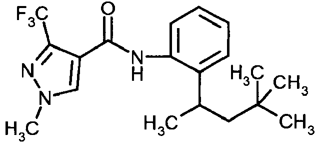 | 100 | 95 |
| 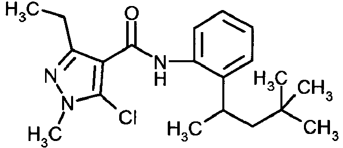 | 100 | 92 |
| 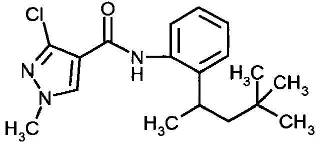 | 100 | 100 |
| 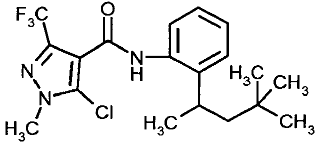 | 100 | 99 |
| 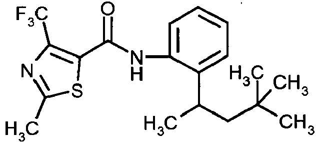 | 100 | 99 |

### Beispiel C

**Botrytis - Test (Bohne) / protektiv**

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

**Tabelle C**

| **Botrytis - Test (Bohne) / protektiv** | | |
|---|---|---|
| Wirkstoff | Aufwandmenge an Wirkstoff in g/ha | Wirkungsgrad in % |
| 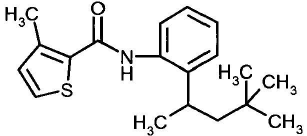 | 500 | 100 |
| 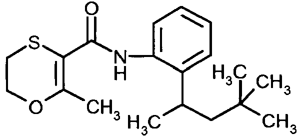 | 500 | 95 |
| 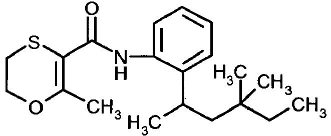 | 500 | 100 |
| 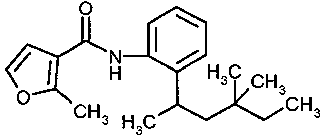 | 500 | 88 |
| 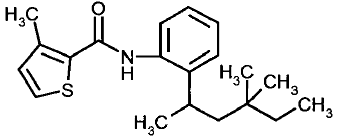 | 500 | 100 |
| 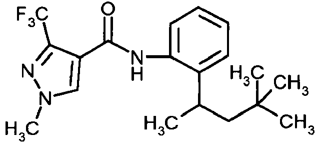 | 500 | 100 |
| 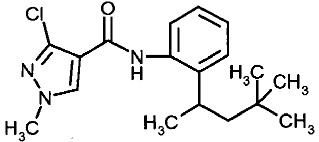 | 500 | 95 |
| 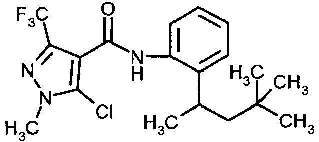 | 500 | 97 |
| 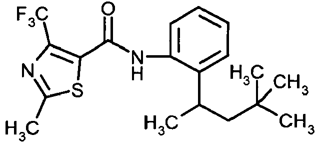 | 500 | 95 |

### Beispiel D

**Puccinia-Test (Weizen) / protektiv**

| | | | |
|---|---|---|---|
| a) | Lösungsmittel: | 50 | Gewichtsteile N,N-Dimethylacetamid |
| | Emulgator: | 1 | Gewichtsteile Alkylarylpolyglykolether |
| b) | Lösungsmittel: | 25 | Gewichtsteile N,N-Dimethylacetamid |
| | Emulgator: | 0,6 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle D**

| **Puccinia-Test (Weizen)** / **protektiv** | | | |
|---|---|---|---|
| Wirkstoff | Aufwandmenge an Wirkstoff in g/ha | Wirkungsgrad in % | Lösungsmittel/ Emulgator |
| 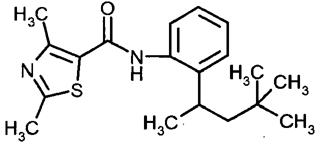 | 500 | 100 | a) |
| 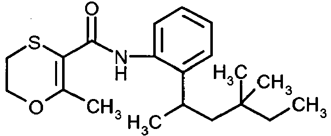 | 500 | 100 | a) |
| 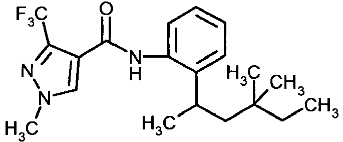 | 500 | 100 | a) |
| 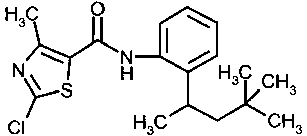 | 500 | 100 | a) |
| 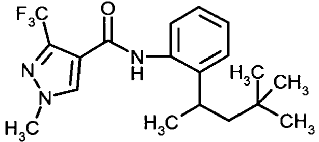 | 500 | 100 | a) |
| 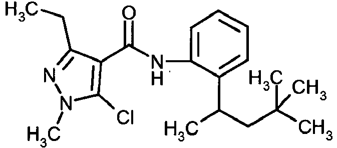 | 500 | 100 | a) |
| 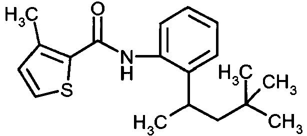 | 500 | 100 | b) |
| 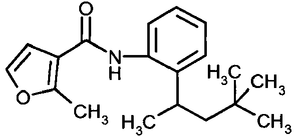 | 500 | 100 | b) |
| 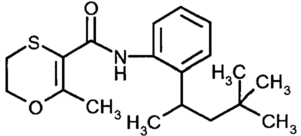 | 500 | 100 | b) |
| 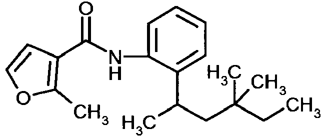 | 500 | 100 | b) |
| | | | |

### Beispiel E

**Sphaerotheca-Test (Gurke) / protektiv**

| | | |
|---|---|---|
| Lösungsmittel: | 49 | Gewichtsteile N, N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von Sphaerotheca fuliginea inokuliert. Anschließend werden die Pflanzen in einem Gewächshaus bei 70 % relativer Luftfeuchtigkeit und einer Temperatur von 23°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle E**

| **Sphaerotheca-Test (Gurke) / protektiv** | | |
|---|---|---|
| Wirkstoff | Aufwandmenge an Wirkstoff in g/ha | Wirkungsgrad in % |
| 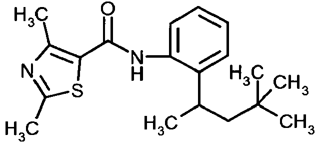 | 750 | 95 |
| 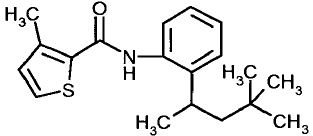 | 750 | 95 |
| 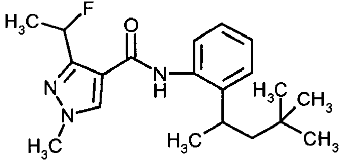 | 750 | 90 |

### Beispiel F

**Erysiphe-Test (Gerste) / protektiv**

| | | |
|---|---|---|
| Lösungsmittel: | 25 | Gewichtsteile N,N-Dimethylacetamid |
| Emulgator: | 0,6 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle F**

| **Erysiphe-Test (Gerste) / protektiv** | | |
|---|---|---|
| Wirkstoff | Aufwandmenge an Wirkstoff in g/ha | Wirkungsgrad in % |
| 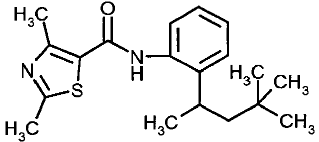 | 500 | 100 |
| 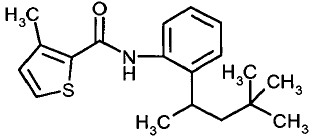 | 500 | 100 |
| 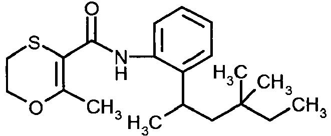 | 500 | 94 |
| 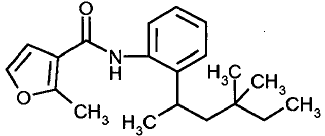 | 500 | 100 |
| 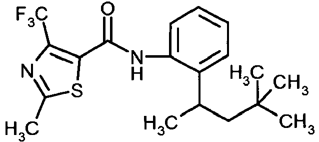 | 500 | 100 |

## Patentansprüche

1. Hexylcarboxanilide der Formel (I) 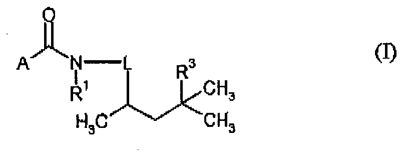 in welcher
L für 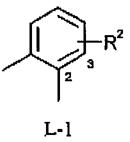
steht, wobei die mit * markierte Bindung mit dem Amid verbunden ist,
R¹ für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁₋C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl₃ (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
(C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogen-alkoxy)carbonyl, (Halogen-C₁-C₄-alkoxy-C₁-C₄-alkyl)carbonyl, (C₃-C₈-Halogen-cycloalkyl)carbonyl mit jeweils 1 bis 9 Fluor-, Chlor- und/oder Bromatomen; oder -C(=O)C(=O)R⁴, -CONR⁵R⁶ oder -CH₂NR⁷R⁸ steht,
R² für Wasserstoff, Fluor, Chlor, Methyl oder Trifluormethyl steht,
R³ für Halogen, C₁-C₈-Alkyl oder C₁-C₈-Halogenalkyl steht,
R⁴ für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
R⁵ und R⁶ unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
R⁵ und R⁶ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁹ enthalten kann,
R⁷ und R⁸ unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
R⁷ und R⁸ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR⁹ enthalten kann,
R⁹ für Wasserstoff oder C₁-C₆-Alkyl steht,
A für den Rest der Formel (A1) 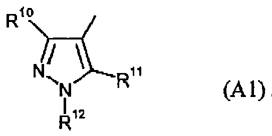 steht, in welcher
R¹⁰ für Wasserstoff, Hydroxy, Formyl, Cyano, Fluor, Chlor, Brom, Nitro, C₁-C₄-Alkyl, C₁-C₄-Alkoxy, C₁-C₄-Alkylthio, C₃-C₆-Cycloalkyl, C₁-C₄-Halogen-alkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht,
R¹¹ für Wasserstoff, Chlor, Brom, Iod, Cyano, C₁-C₄-Alkyl, C₁-C₄-Alkoxy, C₁-C₄-Alkylthio, C₁-C₄-Halogenalkyl oder C₁-C₄-Halogenalkylthio mit jeweils 1 bis 5 Halogenatomen, steht und
R¹² für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder für Phenyl steht.

2. Hexylcarboxanilide der Formel (1) gemäß Anspruch 1, in welcher
L für 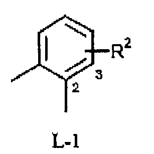 steht,
wobei die mit ^{‡} markierte Bindung mit dem Amid verbunden ist,
R¹ für Wasserstoff, C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₆-Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogen-alkoxy)carbonyl, (Halogen-C₁-C₃-alkoxy-C₁-C₃-alkyl)carbonyl, (C₃-C₆-Halogen-cycloalkyl)carbonyl mit jeweils 1 bis 9 Fluor-, Chlor- und/oder Bromatomen; oder -C(=O)C(=O)R⁴, -CONR⁵R⁶ oder -CH₂NR⁷R⁸ steht,
R² für Wasserstoff, Fluor, Chlor, Methyl oder Trifluormethyl steht,
R³ für Fluor, Chlor, Brom, Iod, C₁-C₆-Alkyl, C₁-C₆-Halogenalkyl mit jeweils 1 bis 13 Fluor,-, Chlor- und/oder Bromatomen steht,
R⁴ für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen steht,
R⁵ und R⁶ unabhängig voneinander jeweils für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen stehen,
R⁵ und R⁶ außerdem gemeinsam mit dem Stickstoffatom, an das sie gebunden sind, einen gegebenenfalls einfach bis vierfach, gleich oder verschieden durch Halogen oder C₁-C₄-Alkyl substituierten gesättigten Heterocyclus mit 5 oder 6 Ringatomen bilden, wobei der Heterocyclus 1 oder 2 weitere, nicht benachbarte Heteroatome aus der Reihe Sauerstoff, Schwefel oder NR⁹ enthalten kann,
R⁷ und R⁸ unabhängig voneinander jeweils für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor- und/oder Bromatomen stehen,
R⁷ und R⁸ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 oder 6 Ringatomen bilden, wobei der Heterocyclus 1 oder 2 weitere, nicht benachbarte Heteroatome aus der Reihe Sauerstoff, Schwefel oder NR⁹ enthalten kann,
R⁹ für Wasserstoff oder C₁-C₄-Alkyl steht,
A für den Rest der Formel (A1) 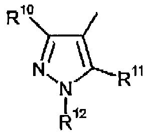 (A1) steht, in welcher
R¹⁰ für Wasserstoff, Hydroxy, Formyl, Cyano, Fluor, Chlor, Brom, Methyl, Ethyl, iso-Propyl, Methoxy, Ethoxy, Methylthio, Ethylthio, Cyclopropyl, C₁-C₂-Halogenalkyl, C₁-C₂-Halogenalkoxy mit jeweils 1 bis 5 Fluor, Chlor und/oder Bromatomen, Trifluormethylthio, Difluormethylthio, Amino-carbonyl, Aminocarbonylmethyl oder Aminocarbonylethylsteht,
R¹¹ für Wasserstoff, Chlor, Brom, Iod, Methyl, Ethyl, Methoxy, Ethoxy, Methylthio, Ethylthio, C₁-C₂-Halogenalkyl mit 1 bis 5 Fluor, Chlor und/oder Bromatomen steht und
R¹² für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl steht.

3. Hexylcarboxanilide der Formel (I) gemäß Anspruch 1 oder 2, in welcher R¹ für Wasserstoff, Formyl oder -C(=O)C(=O)R⁴ steht, wobei R⁴ die in Anspruch 1 oder 2 angegebenen Bedeutungen hat.

4. Hexylcarboxanilide der Formel (I) gemäß Anspruch 1 oder 2, in welcher R³ für Halogen steht.

5. Hexylcarboxanilide der Formel (I) gemäß Anspruch 1 oder 2, in welcher R³ für C₁-C₈-Alkyl steht.

6. Hexylcarboxanilide der Formel (I) gemäß Anspruch 1 oder 2, in welcher R³ für C₁-C₈-Halogenalkyl steht.

7. Verfahren zum Herstellen der Verbindungen der Formel (I) gemäß Anspruch 1, **dadurch gekennzeichnet, dass** man
a) Carbonsäure-Derivate der Formel (II) 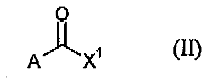 in welcher
A die in Anspruch 1 angegebenen Bedeutungen hat und
X¹ für Halogen oder Hydroxy steht,
mit einem Anilin-Derivate der Formel (III) 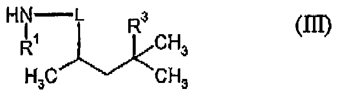 in welcher L, R¹ und R³ die in Anspruch 1 angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt,
oder
b) Hexylcarboxanilide der Fonnel (I-a) 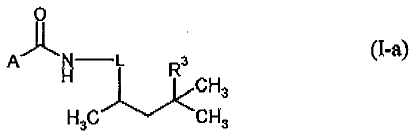 in welcher L, A und R³ die in Anspruch 1 angegebenen Bedeutungen haben mit Halogeniden der Formel (IV)
R^{1-A}-X² (IV)
in welcher
X² für Chlor, Brom oder Iod steht,
R^{1-A} für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)-carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen;
(C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-Alkoxy-C₁-C₄-alkyl)carbonyl, (C₃-C₈-Halogencycloalkyl)carbonyl mit jeweils 1 bis 9 Fluor-, Chlor-
und/oder Bromatomen; oder -C(=O)C(=O)R⁴, -CONR⁵R⁶ oder -CH₂NR⁷R⁸ steht,
wobei R⁴, R⁵, R⁶, R⁷ und R⁸ die in Anspruch 1 angegebenen Bedeutungen haben,
in Gegenwart einer Base und in Gegenwart eines Verdünnungsmittels umsetzt.

8. Mittel zum Bekämpfen unerwünschte Mikroorganismen, **gekennzeichnet durch** einen Gehalt an mindestens einem Hexylcarboxanilid der Formel (I) gemäß Anspruch 1 neben Streckmitteln und/oder oberflächenaktiven Stoffen.

9. Verwendung von Hexylcarboxaniliden der Formel (I) gemäß Anspruch 1 zum Bekämpfen unerwünschter Mikroorganismen im Pflanzenschutz und im Materialschutz.

10. Verfahren zum Bekämpfen unerwünschter Mikroorganismen im Pflanzenschutz und im Materialschutz, **dadurch gekennzeichnet, dass** man Hexylcarboxanilide der Formel (I) gemäß Anspruch 1 auf die Mikroorganismen und/oder deren Lebensraum ausbringt.

11. Verfahren zum Herstellen von Mitteln zum Bekämpfen unerwünschter Mikroorganismen im Pflanzenschutz und im Materialschutz, **dadurch gekennzeichnet, dass** man Hexylcarboxanilide der Formel (I) gemäß Anspruch 1 mit Streckmitteln und/oder oberflächenaktiven Stoffen vermischt.

12. 3-Dichlormethyl-1H-pyrazol-4-carbonsäure-Derivate der Formel (II-a) 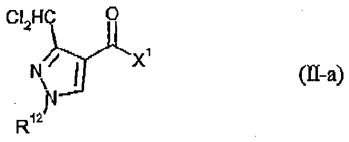 in welcher
R¹² die in Anspruch 1 angegebenen Bedeutungen hat,
X¹ für Halogen oder Hydroxy steht.

13. Verfahren zum Herstellen von 3-Dichlormethyl-1-pyrazol-4-carbonsäure-Derivaten der Formel (II-a) gemäß Anspruch 12, **dadurch gekennzeichnet, dass** man 3-Formyl-1H-pyrazol-4-carbonsäuren der Formel (XI) 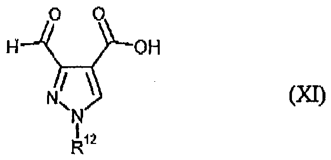 in welcher R¹² die in Anspruch 1 angegebenen Bedeutungen hat,
mit einem Chlorierungsmittel in Gegenwart eines Verdünnungsmittels umsetzt.

14. 3-Dichlormethyl-1H-pyrazol-4-carbonsäureester der Formel (XII) 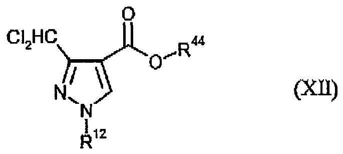 in welcher
R¹² die in Anspruch 1 angegebenen Bedeutungen hat,
R⁴⁴ für C₁-C₄-Alkyl steht.

15. Verfahren zum Herstellen von 3-Dichlormethyl-1H-pyrazol-4-carbonsäureester der Formel (XII) gemäß Anspruch 14, **dadurch gekennzeichnet, dass** man 3-Formyl-1H-pyrazol-4-carbonsäureester der Formel (X) 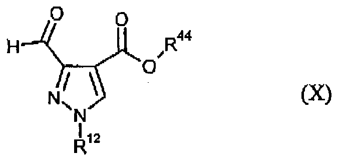 in welcher
R¹² die in Anspruch 1 angegebenen Bedeutungen hat,
R⁴⁴ für C₁-C₄-Alkyl steht,
mit einem Chlorierungsmittel in Gegenwart eines Verdünnungsmittels umsetzt.

## Claims

1. Hexylcarboxanilides of the formula (I) 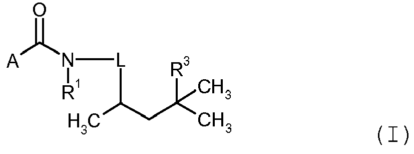 in which
L represents 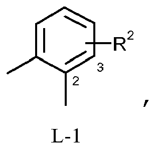
where the bond marked with * is attached to the amide,
R¹ represents hydrogen, C₁-C₈-alkyl, C₁-C₆-alkylsulphinyl, C₁-C₆-alkylsulphonyl, C₁-C₄-alkoxy-C₁-C₄-alkyl, C₃-C₈-cycloalkyl; C₁-C₆-haloalkyl, C₁-C₄-haloalkylthio, C₁-C₄-haloalkylsulphinyl, C₁-C₄-haloalkylsulphonyl, halo-C₁-C₄-alkoxy-C₁-C₄-alkyl, C₃-C₈-halocycloalkyl having in each case 1 to 9 fluorine, chlorine and/or bromine atoms; formyl, formyl-C₁-C₃-alkyl, (C₁-C₃-alkyl) carbonyl-C₁-C₃-alkyl, (C₁-C₃-alkoxy) carbonyl-C₁-C₃-alkyl; halo- (C₁-C₃-alkyl) carbonyl-C₁-C₃-alkyl, halo- (C₁-C₃-alkoxy)carbonyl-C₁-C₃-alkyl having in each case 1 to 13 fluorine, chlorine and/or bromine atoms;
(C₁-C₈-alkyl)carbonyl, (C₁-C₈-alkoxy)carbonyl, (C₁-C₄-alkoxy-C₁-C₄-alkyl) carbonyl, (C₃-C₈-cycloalkyl)carbonyl; (C₁-C₆-haloalkyl)carbonyl, (C₁-C₆-haloalkoxy) carbonyl, (halo-C₁-C₄-alkoxy-C₁-C₄-alkyl)carbonyl, (C₃-C₈-halocycloalkyl)carbonyl having in each case 1 to 9 fluorine, chlorine and/or bromine atoms; or -C(=O)C(=O)R⁴, - CONR⁵R⁶ or -CH₂NR⁷R⁸,
R² represents hydrogen, fluorine, chlorine, methyl or trifluoromethyl,
R³ represents halogen, C₁-C₈-alkyl or C₁-C₈-haloalkyl,
R⁴ represents hydrogen, C₁-C₈-alkyl, C₁-C₈-alkoxy, C₁-C₄-alkoxy-C₁-C₄-alkyl, C₃-C₈-cycloalkyl; C₁-C₆-haloalkyl, C₁-C₆-haloalkoxy, halo-C₁-C₄-alkoxy-C₁-C₄-alkyl, C₃-C₈-halocycloalkyl having in each case 1 to 9 fluorine, chlorine and/or bromine atoms,
R⁵ and R⁶ independently of one another each represent hydrogen, C₁-C₈-alkyl, C₁-C₄-alkoxy-C₁-C₄-alkyl, C₃-C₈-cycloalkyl; C₁-C₈-haloalkyl, halo-C₁-C₄-alkoxy-C₁-C₄-alkyl, C₃-C₈-halocycloalkyl having in each case 1 to 9 fluorine, chlorine and/or bromine atoms,
R⁵ and R⁶ furthermore together with the nitrogen atom to which they are attached form a saturated heterocycle having 5 to 8 ring atoms which is optionally mono- or polysubstituted by identical or different substituents from the group consisting of halogen and C₁-C₄-alkyl, where the heterocycle may contain 1 or 2 further non-adjacent heteroatoms from the group consisting of oxygen, sulphur and NR⁹,
R⁷ and R⁸ independently of one another represent hydrogen, C₁-C₈-alkyl, C₃-C₈-cycloalkyl; C₁-C₈-haloalkyl, C₃-C₈-halocycloalkyl having in each case 1 to 9 fluorine, chlorine and/or bromine atoms,
R⁷ and R⁸ furthermore together with the nitrogen atom to which they are attached form a saturated heterocycle having 5 to 8 ring atoms which is optionally mono- or polysubstituted by identical or different substituents from the group consisting of halogen and C₁-C₄-alkyl, where the heterocycle may contain 1 or 2 further non-adjacent heteroatoms from the group consisting of oxygen, sulphur and NR⁹,
R⁹ represents hydrogen or C₁-C₆-alkyl,
A represents the radical of the formula (A1) 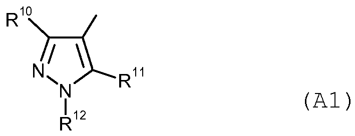 in which
R¹⁰ represents hydrogen, hydroxyl, formyl, cyano, fluorine, chlorine, bromine, nitro, C₁-C₄-alkyl, C₁-C₄-alkoxy, C₁-C₄-alkylthio, C₃-C₆-cycloalkyl, C₁-C₄-haloalkyl, C₁-C₄-haloalkoxy or C₁-C₄-haloalkylthio having in each case 1 to 5 halogen atoms, aminocarbonyl or aminocarbonyl-C₁-C₄-alkyl,
R¹¹ represents hydrogen, chlorine, bromine, iodine, cyano, C₁-C₄-alkyl, C₁-C₄-alkoxy, C₁-C₄-alkylthio, C₁-C₄-haloalkyl or C₁-C₄-haloalkylthio having in each case 1 to 5 halogen atoms, and
R¹² represents hydrogen, C₁-C₄-alkyl, hydroxy-C₁-C₄-alkyl, C₂-C₆-alkenyl, C₃-C₆-cycloalkyl, C₁-C₄-alkylthio-C₁-C₄-alkyl, C₁-C₄-alkoxy-C₁-C₄-alkyl, C₁-C₄-haloalkyl, C₁-C₄-haloalkylthio-C₁-C₄-alkyl, C₁-C₄-haloalkoxy-C₁-C₄-alkyl having in each case 1 to 5 halogen atoms, or represents phenyl.

2. Hexylcarboxanilides of the formula (I) according to Claim 1 in which
L represents 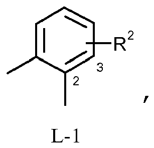
where the bond marked with * is attached to the amide,
R¹ represents hydrogen, C₁-C₆-alkyl, C₁-C₄-alkylsulphinyl, C₁-C₄-alkylsulphonyl, C₁-C₃-alkoxy-C₁-C₃-alkyl, C₃-C₆-cycloalkyl; C₁-C₄-haloalkyl, C₁-C₄-haloalkylthio, C₁-C₄-haloalkylsulphinyl, C₁-C₄-haloalkylsulphonyl, halo-C₁-C₃-alkoxy-C₁-C₃-alkyl, C₃-C₈-halocycloalkyl having in each case 1 to 9 fluorine, chlorine and/or bromine atoms; formyl, formyl-C₁-C₃-alkyl, (C₁-C₃-alkyl) carbonyl-C₁-C₃-alkyl, (C₁-C₃-alkoxy)-carbonyl-C₁-C₃-alkyl; halo-(C₁-C₃-alkyl) carbonyl-C₁-C₃-alkyl, halo- (C₁-C₃-alkoxy) carbonyl-C₁-C₃-alkyl having in each case 1 to 13 fluorine, chlorine and/or bromine atoms;
(C₁-C₆-alkyl)carbonyl, (C₁-C₄-alkoxy) carbonyl, (C₁-C₃-alkoxy-C₁-C₃-alkyl) carbonyl, (C₃-C₆-cycloalkyl)carbonyl; (C₁-C₄-haloalkyl)carbonyl, (C₁-C₄-haloalkoxy) carbonyl, (halo-C₁-C₃-alkoxy-C₁-C₃-alkyl)carbonyl, (C₃-C₆-halocycloalkyl)carbonyl having in each case 1 to 9 fluorine, chlorine and/or bromine atoms; or -C(=O)C(=O)R⁴, - CONR⁵R⁶ or -CH₂NR⁷R⁸,
R² represents hydrogen, fluorine, chlorine, methyl or trifluoromethyl,
R³ represents fluorine, chlorine, bromine, iodine, C₁-C₆-alkyl, C₁-C₆-haloalkyl having in each case 1 to 13 fluorine, chlorine and/or bromine atoms,
R⁴ represents hydrogen, C₁-C₆-alkyl, C₁-C₄-alkoxy, C₁-C₃-alkoxy-C₁-C₃-alkyl, C₃-C₆-cycloalkyl; C₁-C₄-haloalkyl, C₁-C₄-haloalkoxy, halo-C₁-C₃-alkoxy-C₁-C₃-alkyl, C₃-C₆-halocycloalkyl having in each case 1 to 9 fluorine, chlorine and/or bromine atoms,
R⁵ and R⁶ independently of one another each represent hydrogen, C₁-C₆-alkyl, C₁-C₃-alkoxy-C₁-C₃-alkyl, C₃-C₆-cycloalkyl; C₁-C₄-haloalkyl, halo-C₁-C₃-alkoxy-C₁-C₃-alkyl, C₃-C₆-halocycloalkyl having in each case having 1 to 9 fluorine, chlorine and/or bromine atoms,
R⁵ and R⁶ furthermore together with the nitrogen atom to which they are attached form a saturated heterocycle having 5 or 6 ring atoms which is optionally mono- to tetrasubstituted by identical or different substituents from the group consisting of halogen and C₁-C₄-alkyl, where the heterocycle may contain 1 or 2 further non-adjacent heteroatoms from the group consisting of oxygen, sulphur and NR³,
R⁷ and R⁸ independently of one another each represent hydrogen, C₁-C₆-alkyl, C₃-C₆-cycloalkyl; C₁-C₄-haloalkyl, C₃-C₆-halocycloalkyl having in each case 1 to 9 fluorine, chlorine and/or bromine atoms,
R⁷ and R⁸ furthermore together with the nitrogen atom to which they are attached form a saturated heterocycle having 5 or 6 ring atoms which is optionally mono- or polysubstituted by identical or different substituents from the group consisting of halogen and C₁-C₄-alkyl, where the heterocycle may contain 1 or 2 further non-adjacent heteroatoms from the group consisting of oxygen, sulphur and NR³,
R³ represents hydrogen or C₁-C₄-alkyl,
A represents the radical of the formula (A1) 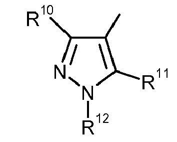 (A1) in which
R¹⁰ represents hydrogen, hydroxyl, formyl, cyano, fluorine, chlorine, bromine, methyl, ethyl, isopropyl, methoxy, ethoxy, methylthio, ethylthio, cyclopropyl, C₁-C₂-haloalkyl, C₁-C₂-haloalkoxy having in each 1 to 5 fluorine, chlorine and/or bromine atoms, trifluoromethylthio, difluoromethylthio, aminocarbonyl, aminocarbonylmethyl or aminocarbonylethyl,
R¹¹ represents hydrogen, chlorine, bromine, iodine, methyl, ethyl, methoxy, ethoxy, methylthio, ethylthio, C₁-C₂-haloalkyl having 1 to 5 fluorine, chlorine and/or bromine atoms and
R¹² represents hydrogen, methyl, ethyl, n-propyl, isopropyl, C₁-C₂-haloalkyl having 1 to 5 fluorine, chlorine and/or bromine atoms, hydroxymethyl, hydroxyethyl, cyclopropyl, cyclopentyl, cyclohexyl or phenyl.

3. Hexylcarboxanilides of the formula (I) according to Claim 1 or 2 in which R¹ represents hydrogen, formyl or -C(=O)C(=O)R⁴, where R⁴ is as defined in Claim 1 or 2.

4. Hexylcarboxanilides of the formula (I) according to Claim 1 or 2 in which R³ represents halogen.

5. Hexylcarboxanilides of the formula (I) according to Claim 1 or 2 in which R³ represents C₁-C₈-alkyl.

6. Hexylcarboxanilides of the formula (I) according to Claim 1 or 2 in which R³ represents C₁-C₈-haloalkyl.

7. Process for preparing the compounds of the formula (I) according to Claim 1, **characterized in that**
a) carboxylic acid derivatives of the formula (II) 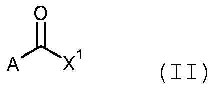 in which
A is as defined in Claim 1 and
X¹ represents halogen or hydroxyl
are reacted with an aniline derivative of the formula (III) 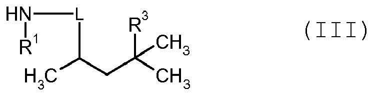 in which L, R¹ and R³ are as defined in Claim 1,
if appropriate in the presence of a catalyst, if appropriate in the presence of a condensing agent, if appropriate in the presence of an acid binder and if appropriate in the presence of a diluent,
or
b) hexylcarboxanilides of the formula (I-a) 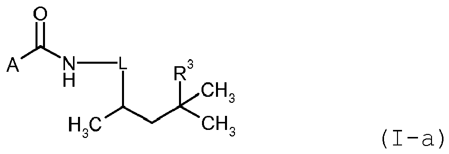 in which L, A and R³ are as defined in Claim 1 are reacted with halides of the formula (IV)
**R^{1-A}-X²** (IV)
in which
X² represents chlorine, bromine or iodine,
R^{1-A} represents C₁-C₈-alkyl, C₁-C₆-alkylsulphinyl, C₁-C₆-alkylsulphonyl, C₁-C₄-alkoxy-C₁-C₄-alkyl, C₃-C₈-cycloalkyl; C₁-C₆-haloalkyl, C₁-C₄-haloalkylthio, C₁-C₄-haloalkylsulphinyl, C₁-C₄-haloalkylsulphonyl, halo-C₁-C₄-alkoxy-C₁-C₄-alkyl, C₃-C₈-halocycloalkyl having in each case 1 to 9 fluorine, chlorine and/or bromine atoms; formyl, formyl-C₁-C₃-alkyl, (C₁-C₃-alkyl) carbonyl-C₁-C₃-alkyl, (C₁-C₃-alkoxy) carbonyl-C₁-C₃-alkyl; halo- (C₁-C₃-alkyl) carbonyl-C₁-C₃-alkyl, halo- (C₁-C₃-alkoxy) carbonyl-C₁-C₃-alkyl having in each case 1 to 13 fluorine, chlorine and/or bromine atoms;
(C₁-C₈-alkyl) carbonyl, (C₁-C₈-alkoxy) carbonyl, (C₁-C₄-alkoxy-C₁-C₄-alkyl) carbonyl, (C₃-C₈-cycloalkyl)carbonyl; (C₁-C₆-haloalkyl)carbonyl, (C₁-C₆-haloalkoxy)carbonyl, (halo-C₁-C₄-alkoxy-C₁-C₄-alkyl) carbonyl, (C₃-C₈-halocycloalkyl)carbonyl having in each case 1 to 9 fluorine, chlorine and/or bromine atoms; or -C(=O)C(=O) R⁴, -CONR⁵R⁶ or -CH₂NR⁷R⁸,
where R⁴, R⁵, R⁶, R⁷ and R⁸ are as defined in Claim 1
in the presence of a base and in the presence of a diluent.

8. Compositions for controlling unwanted microorganisms, **characterized in that** they comprise at least one hexylcarboxanilide of the formula (I) according to Claim 1, in addition to extenders and/or surfactants.

9. Use of hexylcarboxanilides of the formula (I) according to Claim 1 for controlling unwanted microorganisms in crop protection and in the protection of materials.

10. Method for controlling unwanted microorganisms in crop protection and in the protection of materials, **characterized in that** hexylcarboxanilides of the formula (I) according to Claim 1 are applied to the microorganisms and/or their habitats.

11. Process for preparing compositions for controlling unwanted microorganisms in crop protection and in the protection of materials, **characterized in that** hexylcarboxanilides of the formula (I) according to Claim 1 are mixed with extenders and/or surfactants.

12. 3-Dichloromethyl-1H-pyrazole-4-carboxylic acid derivatives of the formula (II-a) 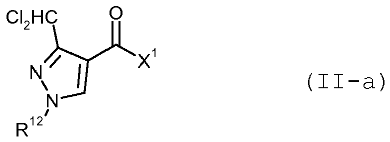 in which
R¹² is as defined in Claim 1,
X¹ represents halogen or hydroxyl.

13. Process for preparing 3-dichloromethyl-1H-pyrazole-4-carboxylic acid derivatives of the formula (II-a) according to Claim 12, **characterized in that** 3-formyl-1H-pyrazole-4-carboxylic acids of the formula (XI) 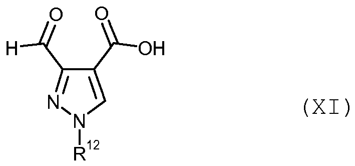 in which R¹² is as defined in Claim 1 are reacted with a chlorinating agent in the presence of a diluent.

14. 3-Dichloromethyl-1H-pyrazole-4-carboxylic acid esters of the formula (XII) 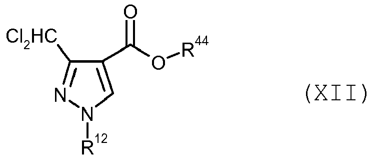 in which
R¹² is as defined in Claim 1,
R⁴⁴ represents C₁-C₄-alkyl.

15. Process for preparing 3-dichloromethyl-1H-pyrazole-4-carboxylic acid esters of the formula (XII) according to Claim 14, **characterized in that** 3-formyl-1H-pyrazole-4-carboxylic acid esters of the formula (X) 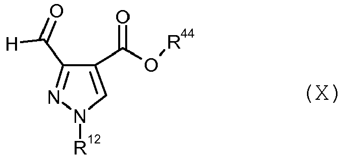 in which
R¹² is as defined in Claim 1,
R⁴⁴ represents C₁-C₄-alkyl
are reacted with a chlorinating agent in the presence of a diluent.

## Revendications

1. Hexylcarboxanilides de formule (I) 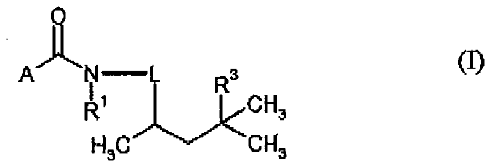 dans laquelle
L représente 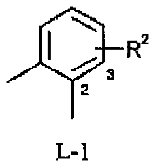
la liaison marquée par * étant rattachée à l'amide,
R¹ représente un atome d'hydrogène, un groupe alkyle en C₁-C₈, alkyl(C₁-C₆)sulfinyle, alkyl(C₁-C₆)-sulfonyle, alcoxy(C₁-C₄)-alkyle(C₁-C₄), cycloalkyle en C₃-C₈, halogénoalkyle(C₁-C₆), halogéno-alkyl(C₁-C₄)thio, halogénoalkyl(C₁-C₄)sulfinyle, halogénoalkyl(C₁-C₄)sulfonyle, halogéno-alcoxy(C₁-C₄)-alkyle(C₁-C₄), halogéno-cycloalkyle(C₃-C₈) comportant chaque fois de 1 à 9 atomes de fluor, chlore et/ou brome ; un groupe formyle, formyl-alkyle(C₁-C₃), (alkyl(C₁-C₃))-carbonyl-alkyle(C₁-C₃), (alcoxy(C₁-C₃))carbonyl-alkyle(C₁-C₃) ; halogéno-(alkyl(C₁-C₃))carbonyl-alkyle(C₁-C₃), halogéno(alcoxy(C₁-C₃))carbonyl-alkyle(C₁-C₃) comportant chaque fois de 1 à 13 atomes de fluor, chlore et/ou brome ; un groupe (alkyl (C₁-C₈)) carbonyle, (alcoxy(C₁-C₈))-carbonyle, (alcoxy(C₁-C₄)-alkyl(C₁-C₄))carbonyle, (cycloalkyl(C₃-C₈))carbonyle ; (halogéno-alkyl(C₁-C₆))carbonyle, halogénoalcoxy(C₁-C₆))-carbonyle, (halogénoalcoxy(C₁-C₄)-alkyl(C₁-C₄))-carbonyle, (halogénocycloalkyl(C₃-C₈))carbonyle comportant chaque fois de 1 à 9 atomes de fluor, chlore et/ou brome ; ou -C(=O)C(=O)R⁴, -CONR⁵R⁶ ou -CH²NR⁷R⁸,
R² représente un atome d'hydrogène, de fluor, chlore, le groupe méthyle ou trifluorométhyle,
R³ représente un atome d'halogène, un groupe alkyle en C₁-C₈ ou halogénoalkyle(C₁-C₈),
R⁴ représente un atome d'hydrogène, un groupe alkyle en C₁-C₈, alcoxy en C₁-C₈, alcoxy(C₁-C₄)-alkyle(C₁-C₄), cycloalkyle en C₃-C₈ ; halogénoalkyle(C₁-C₆), halogénoalcoxy(C₁-C₆), halogénoalcoxy(C₁-C₄)-alkyle(C₁-C₄), halogéno-cycloalkyle(C₃-C₈) comportant chaque fois de 1 à 9 atomes de fluor, chlore et/ou brome,
R⁵ et R⁶ représentent chacun, indépendamment l'un de l'autre, un atome d'hydrogène, un groupe alkyle en C₁-C₈, alcoxy(C₁-C₄)-alkyle(C₁-C₄), cycloalkyle en C₃-C₈ ; halogénoalkyle(C₁-C₈), halogéno-alcoxy(C₁-C₄)-alkyle(C₁-C₄), halogéno-cycloalkyle(C₃-C₈) comportant chaque fois de 1 à 9 atomes de fluor, chlore et/ou brome,
R⁵ et R⁶ en outre forment ensemble avec l'atome d'azote, auquel ils sont liés, un hétérocycle saturé ayant de 5 à 8 atomes formant le cycle, portant éventuellement un ou plusieurs substituants, identiques ou différents, halogéno ou alkyle en C₁-C₄, l'hétérocycle pouvant contenir 1 ou 2 autres hétéroatomes non contigus, choisis dans la série constituée par les atomes d'oxygène, de soufre ou NR⁹,
R⁷ et R⁸ représentent indépendamment l'un de l'autre un atome d'hydrogène, un groupe alkyle en C₁-C₈, cycloalkyle en C₃-C₈ ; halogénoalkyle(C₁-C₈), halogénocycloalkyle(C₃-C₈) comportant chaque fois de 1 à 9 atomes de fluor, chlore et/ou brome,
R⁷ et R⁸ en outre forment ensemble avec l'atome d'azote, auquel ils sont liés, un hétérocycle saturé ayant de 5 à 8 atomes formant le cycle, portant éventuellement un ou plusieurs substituants, identiques ou différents, halogéno ou alkyle en C₁-C₄, l'hétérocycle pouvant contenir 1 ou 2 autres hétéroatomes non contigus, choisis dans la série constituée par les atomes d'oxygène, de soufre ou NR⁹,
R⁹ représente un atome d'hydrogène ou un groupe alkyle en C₁-C₆,
A représente le radical de formule (A1) 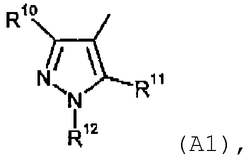 dans laquelle
R¹⁰ représente un atome d'hydrogène, le groupe hydroxy, formyle, cyano, un atome de fluor, chlore, brome, un groupe nitro, alkyle en C₁-C₄, alcoxy en C₁-C₄, alkyl(C₁-C₄)hio, cycloalkyle en C₃-C₆, halogénoalkyle(C₁-C₄), halogénoalcoxy(C₁-C₄) ou halogénoalkyl(C₁-C₄)-thio comportant chaque fois de 1 à 5 atomes d'halogène, aminocarbonyle ou aminocarbonyl-alkyle(C₁-C₄),
R¹¹ représente un atome d'hydrogène, de chlore, de brome, d'iode, un groupe cyano, alkyle en C₁-C₄, alcoxy en C₁-C₄, alkyl(C₁-C₄)thio, halogénoalkyle(C₁-C₄) ou halogénoalkyl(C₁-C₄)-thio comportant chaque fois de 1 à 5 atomes d'halogène, et
R¹² représente un atome d'hydrogène, un groupe alkyle en C₁-C₄, hydroxyalkyle(C₁-C₄), alcényle en C₂-C₆, cycloalkyle en C₃-C₆, alkyl(C₁-C₄)thio-alkyle(C₁-C₄), alcoxy(C₁-C₄)-alkyle(C₁-C₄), halogénoalkyle(C₁-C₄), halogénoalkyl(C₁-C₄)thio-alkyle(C₁-C₄), halogénoalcoxy(C₁-C₄)-alkyle(C₁-C₄) comportant chaque fois de 1 à 5 atomes d'halogène, ou représente le groupe phényle.

2. Hexylcarboxanilides de formule (I) selon la revendication 1, dans lesquels
L représente 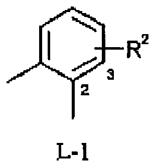
la liaison marquée par * étant rattachée à l'amide,
R¹ représente un atome d'hydrogène, un groupe alkyle en C₁-C₆, alkyl(C₁-C₄)sulfinyle, alkyl (C₁-C₄)-sulfonyle, alcoxy(C₁-C₃)-alkyle(C₁-C₃), cycloalkyle en C₃-C₆, halogénoalkyle(C₁-C₄), halogéno-alkyl(C₁-C₄)thio, halogénoalkyl(C₁-C₄)sulfinyle, halogénoalkyl(C₁-C₄)sulfonyle, halogéno-alcoxy(C₁-C₃)-alkyle(C₁-C₃), halogéno-cycloalkyle(C₃-C₈) comportant chaque fois de 1 à 9 atomes de fluor, chlore et/ou brome ; un groupe formyle, formyl-alkyle(C₁-C₃), (alkyl(C₁-C₃))-carbonyl-alkyle(C₁-C₃), (alcoxy(C₁₋C₃))carbonyl-alkyle(C₁-C₃) ; halogéno-(alkyl(C₁-C₃))carbonyl-alkyle (C₁-C₃), halogéno(alcoxy(C₁-C₃))carbonyl-alkyle(C₁-C₃) comportant chaque fois de 1 à 13 atomes de fluor, chlore et/ou brome ; un groupe (alkyl(C₁-C₆))carbonyle, (alcoxy(C₁-C₄))-carbonyle, (alcoxy(C₁-C₃)-alkyl(C₁-C₃))carbonyle, (cycloalkyl(C₃-C₆))carbonyle ; (halogéno-alkyl(C₁-C₄))carbonyle, halogénoalcoxy(C₁-C₄))-carbonyle, (halogénoalcoxy(C₁-C₃)-alkyl(C₁-C₃))-carbonyle, (halogénocycloalkyl(C₃-C₆))carbonyle comportant chaque fois de 1 à 9 atomes de fluor, chlore et/ou brome ; ou -C(=O)C(=O)R⁴, -CONR⁵R⁶ ou -CH₂NR⁷R⁸,
R² représente un atome d'hydrogène, de fluor, chlore, le groupe méthyle ou trifluorométhyle,
R³ représente un atome de fluor, de chlore, de brome, d'iode, un groupe alkyle en C₁-C₆, halogénoalkyle(C₁-C₆) comportant chaque fois de 1 à 13 atomes de fluor, chlore et/ou brome,
R⁴ représente un atome d'hydrogène, un groupe alkyle en C₁-C₆, alcoxy en C₁-C₄, alcoxy(C₁-C₃)-alkyle(C₁-C₃), cycloalkyle en C₃-C₆ ; halogénoalkyle(C₁-C₄), halogénoalcoxy(C₁-C₄), halogénoalcoxy(C₁-C₃)-alkyle(C₁-C₃), halogéno-cycloalkyle(C₃-C₆) comportant chaque fois de 1 à 9 atomes de fluor, chlore et/ou brome,
R⁵ et R⁶ représentent chacun, indépendamment l'un de l'autre, un atome d'hydrogène, un groupe alkyle en C₁-C₆, alcoxy(C₁-C₃)-alkyle(C₁-C₃), cycloalkyle en C₃-C₆ ; halogénoalkyle(C₁-C₄), halogéno-alcoxy(C₁-C₃)-alkyle(C₁-C₃), halogéno-cycloalkyle(C₃-C₆) comportant chaque fois de 1 à 9 atomes de fluor, chlore et/ou brome,
R⁵ et R⁶ en outre forment ensemble avec l'atome d'azote, auquel ils sont liés, un hétérocycle saturé ayant 5 ou 6 atomes formant le cycle, portant éventuellement de un à quatre substituants, identiques ou différents, halogéno ou alkyle en C₁-C₄, l'hétérocycle pouvant contenir 1 ou 2 autres hétéroatomes non contigus, choisis dans la série constituée par les atomes d'oxygène, de soufre ou NR⁹,
R⁷ et R⁸ représentent indépendamment l'un de l'autre un atome d'hydrogène, un groupe alkyle en C₁-C₆, cycloalkyle en C₃-C₆ ; halogénoalkyle(C₁-C₄), halogénocycloalkyle(C₃-C₆) comportant chaque fois de 1 à 9 atomes de fluor, chlore et/ou brome,
R⁷ et R⁸ en outre forment ensemble avec l'atome d'azote, auquel ils sont liés, un hétérocycle saturé ayant 5 ou 6 atomes formant le cycle, portant éventuellement un ou plusieurs substituants, identiques ou différents, halogéno ou alkyle en C₁-C₄, l'hétérocycle pouvant contenir 1 ou 2 autres hétéroatomes non contigus, choisis dans la série constituée par les atomes d'oxygène, de soufre ou NR⁹,
R⁹ représente un atome d'hydrogène ou un groupe alkyle en C₁-C₄,
A représente le radical de formule (A1) 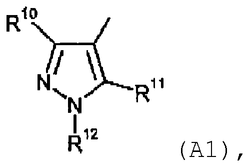 dans laquelle
R¹⁰ représente un atome d'hydrogène, le groupe hydroxy, formyle, cyano, un atome de fluor, chlore, brome, un groupe méthyle, éthyle, isopropyle, méthoxy, éthoxy, méthylthio, éthylthio, cyclopropyle, halogéno-alkyle(C₁-C₂), halogénoalcoxy(C₁-C₂) comportant chaque fois de 1 à 5 atomes de fluor, chlore et/ou brome, trifluorométhylthio, difluorométhylthio, aminocarbonyle, aminocarbonylméthyle ou aminocarbonyléthyle,
R¹¹ représente un atome d'hydrogène, de chlore, de brome, d'iode, un groupe méthyle, éthyle, méthoxy, éthoxy, méthylthio, éthylthio, halogénoalkyle(C₁-C₂) comportant de 1 à 5 atomes de fluor, chlore et/ou brome et
R¹² représente un atome d'hydrogène, un groupe méthyle, éthyle, n-propyle, isopropyle, halogénoalkyle(C₁-C₂) comportant de 1 à 5 atomes de fluor, chlore et/ou brome, le groupe hydroxyméthyle, hydroxyéthyle, cyclopropyle, cyclopentyle, cyclohexyle ou phényle.

3. Hexylcarboxanilides de formule (I) selon la revendication 1 ou 2, dans lesquels R¹ représente un atome d'hydrogène, le groupe formyle ou -C(=O)C(=O)R⁴, R⁴ ayant les significations indiquées dans la revendication 1 ou 2.

4. Hexylcarboxanilides de formule (I) selon la revendication 1 ou 2, dans lesquels R³ représente un atome d'halogène.

5. Hexylcarboxanilides de formule (I) selon la revendication 1 ou 2, dans lesquels R³ représente un groupe alkyle en C₁-C₈.

6. Hexylcarboxanilides de formule (I) selon la revendication 1 ou 2, dans lesquels R³ représente un groupe halogénoalkyle en C₁-C₈.

7. Procédé pour la préparation des composés de formule (I) selon la revendication 1, **caractérisé en ce que**
a) on fait réagir des dérivés d'acides carboxyliques de formule (II) 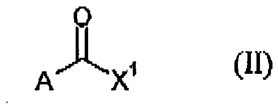 dans laquelle
A a les significations indiquées dans la revendication 1 et
X¹ représente un atome d'halogène ou le groupe hydroxy,
avec un dérivé d'aniline de formule (III) 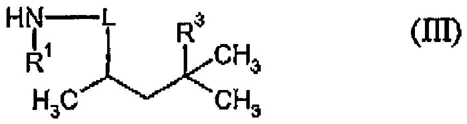 dans laquelle L, R¹ et R³ ont les significations indiquées dans la revendication 1,
éventuellement en présence d'un catalyseur, éventuellement en présence d'un agent de condensation, éventuellement en présence d'un capteur d'acide et éventuellement en présence d'un diluant,
ou
b) on fait réagir des hexylcarboxanilides de formule (I-a) 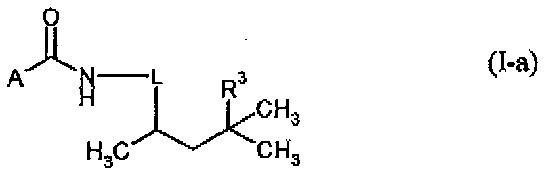 dans laquelle L, A et R³ ont les significations indiquées dans la revendication 1
avec des halogénures de formule (IV)
**R^{1-A}-X²** **(IV)**
dans laquelle
X² représente un atome de chlore, de brome ou d'iode,
R^{1-A} représente un groupe alkyle en C₁-C₈, alkyl (C₁-C₆) sulfinyle, alkyl (C₁-C₆) sulfonyle, alcoxy (C₁-C₄)-alkyle(C₁-C₄), cycloalkyle en C₃-C₈, halogénoalkyle (C₁-C₆), halogéno-alkyl(C₁-C₄)thio, halogénoalkyl(C₁-C₄)-sulfinyle, halogénoalkyl(C₁-C₄)sulfonyle, halogénoalcoxy (C₁-C₄)-alkyle(C₁-C₄), halogéno-cycloalkyle(C₃-C₈) comportant chaque fois de 1 à 9 atomes de fluor, chlore et/ou brome ; un groupe formyle, formyl-alkyle(C₁-C₃), (alkyl(C₁-C₃))carbonyl-alkyle(C₁-C₃), (alcoxy(C₁-C₃))carbonyl-alkyle(C₁-C₃) ; halogéno-(alkyl(C₁-C₃))carbonyl-alkyle(C₁-C₃), halogéno (alcoxy(C₁-C₃))carbonyl-alkyle(C₁-C₃) comportant chaque fois de 1 à 13 atomes de fluor, chlore et/ou brome ;
un groupe (alkyl(C₁-C₈))carbonyle, (alcoxy(C₁-C₆)carbonyle, (alcoxy(C₁-C₄)-alkyl(C₁-C₄))carbonyle, (cycloalkyl(C₃-C₈)-carbonyle ; (halogénoalkyl(C₁-C₆))carbonyle, halogénoalcoxy(C₁-C₆))carbonyle, (halogéno-alcoxy(C₁-C₄)-alkyl(C₁-C₄))carbonyle, (halogénocycloalkyl(C₃-C₈))carbonyle comportant chaque fois de 1 à 9 atomes de fluor, chlore et/ou brome ; ou -C(=O)C(=O)R⁴, -CONR⁵R⁶ ou -CH₂NR⁷R⁸,
R⁴, R⁵, R⁶, R⁷ et R⁸ ayant les significations indiquées dans la revendication 1,
en présence d'une base et en présence d'un diluant.

8. Composition destinée à la lutte contre des micro-organismes indésirables, **caractérisée par** une teneur en au moins un hexylcarboxanilide de formule (I) selon la revendication 1, en plus d'excipients et/ou de substances tensioactives.

9. Utilisation d'hexylcarboxanilides de formule (I) selon la revendication 1, pour la lutte contre des micro-organismes indésirables dans la protection des plantes et dans la protection des matériaux.

10. Procédé pour la lutte contre des micro-organismes indésirables dans la protection des plantes et dans la protection des matériaux, **caractérisé en ce qu'**on applique des hexylcarboxanilides de formule (I) selon la revendication 1 sur les micro-organismes et/ou leur habitat.

11. Procédé pour la fabrication de compositions destinées à la lutte contre des micro-organismes indésirables dans la protection des plantes et dans la protection des matériaux, **caractérisé en ce qu'**on mélange des hexylcarboxanilides de formule (I) selon la revendication 1 avec des excipients et/ou des substances tensioactives.

12. Dérivés d'acide 3-dichorométhyl-1H-pyrazole-4-carboxylique de formule (II-a) 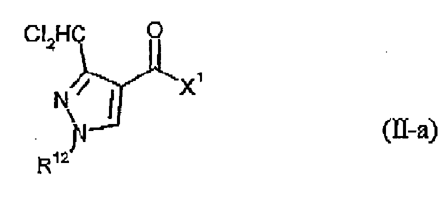 dans laquelle
R¹² a les significations indiquées dans la revendication 1,
X¹ représente un atome d'halogène ou le groupe hydroxy.

13. Procédé pour la préparation de dérivés d'acide 3-dichlorométhyl-1H-pyrazole-4-carboxylique de formule (II-a) selon la revendication 12, **caractérisé en ce qu'**on fait réagir des acides 3-formyl-1H-pyrazole-4-carboxyliques de formule (XI), 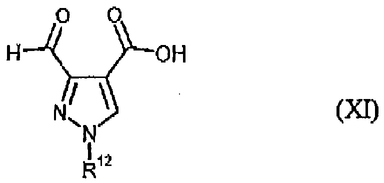 dans laquelle R¹² a les significations indiquées dans la revendication 1,
avec un agent de chloration en présence d'un diluant.

14. Ester d'acide 3-dichlorométhyl-1H-pyrazole-4-carboxylique de formule (XII), 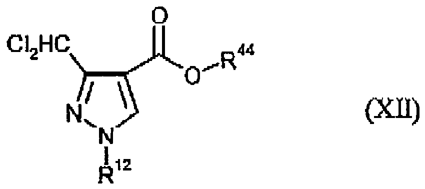 dans laquelle
R¹² a les significations indiquées dans la revendication 1,
R⁴⁴ représente un groupe alkyle en C₁-C₄.

15. Procédé pour la préparation d'ester d'acide 3-dichlorométhyl-1H-pyrazole-4-carboxylique de formule (XII) selon la revendication 14, **caractérisé en ce qu'**on fait réagir un ester d'acide 3-formyl-1H-pyrazole-4-carboxylique de formule (X) 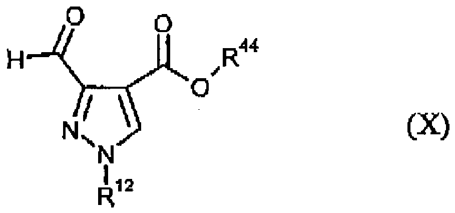 dans laquelle
R¹² a les significations indiquées dans la revendication 1,
R⁴⁴ représente un groupe alkyle en C₁-C₄,
avec un agent de chloration en présence d'un diluant.
